# EUROPEAN PATENT APPLICATION

(11) **EP 4 043 562 A1**
(43) Date of publication of application: **17.08.2022**
(21) Application number: 21157346.4
(22) Date of filing: 16.02.2021
(51) Int. Cl.: C12N 9/16, A61K 38/46

(54) **MUTATED ARYLSULFATASE A WITH INCREASED STABILITY**

(71) Applicant: Rheinische Friedrich-Wilhelms-Universität Bonn, 53113 Bonn (DE)
(72) Inventor: Matzner, Ulrich, 53113 Bonn (DE); Gieselmann, Volkmar, 53347 Bonn-Alfter (DE)
(74) Representative: Kuttenkeuler, David

(57) **Abstract**

The invention is based on the introduction of mutations into the amino acid sequence of human Arylsulfatase A (ASA or ARSA) in order to increase protein stability. The invention introduces amino acid mutations, such as deletions, substitutions or additions, into the C-terminal part of the human ARSA enzyme, in particular at a position around or at amino acid 424, which result in a sequence that does not comprise E424. Provided are further nucleic acids and vectors for the expression of the mutated ARSA of the invention, recombinant cells and pharmaceutical composition comprising the mutated ARSA, as well as its use in the treatment of diseases that are characterized by a reduced activity of endogenous ARSA.

SEQUENCE LISTING

<110>  Rheinische Friedrich-Wilhelms-Universität Bonn

<120>  MUTATED ARYLSULFATASE A WITH INCREASED STABILITY

<130>  U31026EP

<160>  8

<170>  PatentIn version 3.5

<210>  1
<211>  507
<212>  PRT
<213>  Homo sapiens

<400>  1

Met Gly Ala Pro Arg Ser Leu Leu Leu Ala Leu Ala Ala Gly Leu Ala
1               5                   10                  15

Val Ala Arg Pro Pro Asn Ile Val Leu Ile Phe Ala Asp Asp Leu Gly
            20                  25                  30

Tyr Gly Asp Leu Gly Cys Tyr Gly His Pro Ser Ser Thr Thr Pro Asn
        35                  40                  45

Leu Asp Gln Leu Ala Ala Gly Gly Leu Arg Phe Thr Asp Phe Tyr Val
    50                  55                  60

Pro Val Ser Leu Cys Thr Pro Ser Arg Ala Ala Leu Leu Thr Gly Arg
65                  70                  75                  80

Leu Pro Val Arg Met Gly Met Tyr Pro Gly Val Leu Val Pro Ser Ser
                85                  90                  95

Arg Gly Gly Leu Pro Leu Glu Glu Val Thr Val Ala Glu Val Leu Ala
            100                 105                 110

Ala Arg Gly Tyr Leu Thr Gly Met Ala Gly Lys Trp His Leu Gly Val
        115                 120                 125

Gly Pro Glu Gly Ala Phe Leu Pro Pro His Gln Gly Phe His Arg Phe
    130                 135                 140

Leu Gly Ile Pro Tyr Ser His Asp Gln Gly Pro Cys Gln Asn Leu Thr
145                 150                 155                 160

Cys Phe Pro Pro Ala Thr Pro Cys Asp Gly Gly Cys Asp Gln Gly Leu
                165                 170                 175

Val Pro Ile Pro Leu Leu Ala Asn Leu Ser Val Glu Ala Gln Pro Pro
        180                 185                 190

Trp Leu Pro Gly Leu Glu Ala Arg Tyr Met Ala Phe Ala His Asp Leu
    195                 200                 205

Met Ala Asp Ala Gln Arg Gln Asp Arg Pro Phe Phe Leu Tyr Tyr Ala
    210                 215                 220

Ser His His Thr His Tyr Pro Gln Phe Ser Gly Gln Ser Phe Ala Glu
225                 230                 235                 240

Arg Ser Gly Arg Gly Pro Phe Gly Asp Ser Leu Met Glu Leu Asp Ala
                245                 250                 255

Ala Val Gly Thr Leu Met Thr Ala Ile Gly Asp Leu Gly Leu Leu Glu
            260                 265                 270

Glu Thr Leu Val Ile Phe Thr Ala Asp Asn Gly Pro Glu Thr Met Arg
        275                 280                 285

Met Ser Arg Gly Gly Cys Ser Gly Leu Leu Arg Cys Gly Lys Gly Thr
    290                 295                 300

Thr Tyr Glu Gly Gly Val Arg Glu Pro Ala Leu Ala Phe Trp Pro Gly
305                 310                 315                 320

His Ile Ala Pro Gly Val Thr His Glu Leu Ala Ser Ser Leu Asp Leu
                325                 330                 335

Leu Pro Thr Leu Ala Ala Leu Ala Gly Ala Pro Leu Pro Asn Val Thr
            340                 345                 350

Leu Asp Gly Phe Asp Leu Ser Pro Leu Leu Leu Gly Thr Gly Lys Ser
        355                 360                 365

Pro Arg Gln Ser Leu Phe Phe Tyr Pro Ser Tyr Pro Asp Glu Val Arg
    370                 375                 380

Gly Val Phe Ala Val Arg Thr Gly Lys Tyr Lys Ala His Phe Phe Thr
385                 390                 395                 400

Gln Gly Ser Ala His Ser Asp Thr Thr Ala Asp Pro Ala Cys His Ala
                405                 410                 415

Ser Ser Ser Leu Thr Ala His Glu Pro Pro Leu Leu Tyr Asp Leu Ser
            420                 425                 430

Lys Asp Pro Gly Glu Asn Tyr Asn Leu Leu Gly Gly Val Ala Gly Ala
        435                 440                 445

Thr Pro Glu Val Leu Gln Ala Leu Lys Gln Leu Gln Leu Leu Lys Ala
    450                 455                 460

Gln Leu Asp Ala Ala Val Thr Phe Gly Pro Ser Gln Val Ala Arg Gly
465                 470                 475                 480

Glu Asp Pro Ala Leu Gln Ile Cys Cys His Pro Gly Cys Thr Pro Arg
                485                 490                 495

Pro Ala Cys Cys His Cys Pro Asp Pro His Ala
            500                 505

<210>  2
<211>  1524
<212>  DNA
<213>  Homo sapiens

<400>  2
atgggggcac cgcggtccct cctcctggcc ctggctgctg gcctggccgt tgcccgtccg      60

cccaacatcg tgctgatctt tgccgacgac ctcggctatg gggacctggg ctgctatggg     120

caccccagct ctaccactcc caacctggac cagctggcgg cgggagggct gcggttcaca     180

gacttctacg tgcctgtgtc tctgtgcaca ccctctaggg ccgccctcct gaccggccgg     240

ctcccggttc ggatgggcat gtaccctggc gtcctggtgc ccagctcccg ggggggcctg     300

cccctggagg aggtgaccgt ggccgaagtc ctggctgccc gaggctacct cacaggaatg     360

gccggcaagt ggcaccttgg ggtggggcct gagggggcct tcctgccccc ccatcagggc     420

ttccatcgat ttctaggcat cccgtactcc cacgaccagg gcccctgcca gaacctgacc     480

tgcttcccgc cggccactcc ttgcgacggt ggctgtgacc agggcctggt ccccatccca     540

ctgttggcca acctgtccgt ggaggcgcag cccccctggc tgcccggact agaggcccgc     600

tacatggctt tcgcccatga cctcatggcc gacgcccagc gccaggatcg ccccttcttc     660

ctgtactatg cctctcacca cacccactac cctcagttca gtgggcagag ctttgcagag     720

cgttcaggcc gcgggccatt tggggactcc ctgatggagc tggatgcagc tgtggggacc     780

ctgatgacag ccatagggga cctggggctg cttgaagaga cgctggtcat cttcactgca     840

gacaatggac ctgagaccat gcgtatgtcc cgaggcggct gctccggtct cttgcggtgt     900

ggaaagggaa cgacctacga gggcggtgtc cgagagcctg ccttggcctt ctggccaggt     960

catatcgctc ccggcgtgac ccacgagctg gccagctccc tggacctgct gcctaccctg    1020

gcagccctgg ctggggcccc actgcccaat gtcaccttgg atggctttga cctcagcccc    1080

ctgctgctgg gcacaggcaa gagccctcgg cagtctctct tcttctaccc gtcctaccca     1140

gacgaggtcc gtggggtttt tgctgtgcgg actggaaagt acaaggctca cttcttcacc     1200

cagggctctg cccacagtga taccactgca gaccctgcct gccacgcctc cagctctctg     1260

actgctcatg agcccccgct gctctatgac ctgtccaagg accctggtga gaactacaac     1320

ctgctggggg gtgtggccgg ggccacccca gaggtgctgc aagccctgaa acagcttcag     1380

ctgctcaagg cccagttaga cgcagctgtg accttcggcc ccagccaggt ggcccggggc     1440

gaggaccccg ccctgcagat ctgctgtcat cctggctgca ccccccgccc agcttgctgc     1500

cattgcccag atccccatgc ctga                                            1524

<210>  3
<211>  507
<212>  PRT
<213>  artificial

<220>
<223>  mutated human ARSA with increased enzymatic activity

<400>  3

Met Gly Ala Pro Arg Ser Leu Leu Leu Ala Leu Ala Ala Gly Leu Ala
1               5                   10                  15

Val Ala Arg Pro Pro Asn Ile Val Leu Ile Phe Ala Asp Asp Leu Gly
            20                  25                  30

Tyr Gly Asp Leu Gly Cys Tyr Gly His Pro Ser Ser Thr Thr Pro Asn
        35                  40                  45

Leu Asp Gln Leu Ala Ala Gly Gly Leu Arg Phe Thr Asp Phe Tyr Val
    50                  55                  60

Pro Val Ser Leu Cys Thr Pro Ser Arg Ala Ala Leu Leu Thr Gly Arg
65                  70                  75                  80

Leu Pro Val Arg Met Gly Met Tyr Pro Gly Val Leu Val Pro Ser Ser
                85                  90                  95

Arg Gly Gly Leu Pro Leu Glu Glu Val Thr Val Ala Glu Val Leu Ala
            100                 105                 110

Ala Arg Gly Tyr Leu Thr Gly Met Ala Gly Lys Trp His Leu Gly Val
        115                 120                 125

Gly Pro Glu Gly Ala Phe Leu Pro Pro His Gln Gly Phe His Arg Phe
    130                 135                 140

Leu Gly Ile Pro Tyr Ser His Asp Gln Gly Pro Cys Gln Asn Leu Thr
145                 150                 155                 160

Cys Phe Pro Pro Ala Thr Pro Cys Asp Gly Gly Cys Asp Gln Gly Leu
                165                 170                 175

Val Pro Ile Pro Leu Leu Ala Asn Leu Ser Val Glu Ala Gln Pro Pro
            180                 185                 190

Trp Leu Pro Gly Leu Glu Ala Arg Tyr Val Ala Phe Ala His Asp Leu
        195                 200                 205

Met Ala Asp Ala Gln Arg Gln Asp Arg Pro Phe Phe Leu Tyr Tyr Ala
    210                 215                 220

Ser His His Thr His Tyr Pro Gln Phe Ser Gly Gln Ser Phe Ala Glu
225                 230                 235                 240

Arg Ser Gly Arg Gly Pro Phe Gly Asp Ser Leu Met Glu Leu Asp Ala
                245                 250                 255

Ala Val Gly Thr Leu Met Thr Ala Ile Gly Asp Leu Gly Leu Leu Glu
            260                 265                 270

Glu Thr Leu Val Ile Phe Thr Ala Asp Asn Gly Pro Glu Leu Met Arg
        275                 280                 285

Met Ser Asn Gly Gly Cys Ser Gly Leu Leu Arg Cys Gly Lys Gly Thr
    290                 295                 300

Thr Tyr Glu Gly Gly Val Arg Glu Pro Ala Leu Ala Phe Trp Pro Gly
305                 310                 315                 320

His Ile Ala Pro Gly Val Thr His Glu Leu Ala Ser Ser Leu Asp Leu
                325                 330                 335

Leu Pro Thr Leu Ala Ala Leu Ala Gly Ala Pro Leu Pro Asn Val Thr
            340                 345                 350

Leu Asp Gly Phe Asp Leu Ser Pro Leu Leu Leu Gly Thr Gly Lys Ser
        355                 360                 365

Pro Arg Gln Ser Leu Phe Phe Tyr Pro Ser Tyr Pro Asp Glu Val Arg
    370                 375                 380

Gly Val Phe Ala Val Arg Thr Gly Lys Tyr Lys Ala His Phe Phe Thr
385                 390                 395                 400

Gln Gly Ser Ala His Ser Asp Thr Thr Ala Asp Pro Ala Cys His Ala
                405                 410                 415

Ser Ser Ser Leu Thr Ala His Glu Pro Pro Leu Leu Tyr Asp Leu Ser
            420                 425                 430

Lys Asp Pro Gly Glu Asn Tyr Asn Leu Leu Gly Gly Val Ala Gly Ala
        435                 440                 445

Thr Pro Glu Val Leu Gln Ala Leu Lys Gln Leu Gln Leu Leu Lys Ala
    450                 455                 460

Gln Leu Asp Ala Ala Val Thr Phe Gly Pro Ser Gln Val Ala Arg Gly
465                 470                 475                 480

Glu Asp Pro Ala Leu Gln Ile Cys Cys His Pro Gly Cys Thr Pro Arg
                485                 490                 495

Pro Ala Cys Cys His Cys Pro Asp Pro His Ala
            500                 505

<210>  4
<211>  507
<212>  PRT
<213>  artificial

<220>
<223>  mutated human ARSA with increased enzymatic activity and
       stability

<220>
<221>  misc_feature
<222>  (424)..(424)
<223>  Xaa can be any naturally occurring amino acid

<400>  4

Met Gly Ala Pro Arg Ser Leu Leu Leu Ala Leu Ala Ala Gly Leu Ala
1               5                   10                  15

Val Ala Arg Pro Pro Asn Ile Val Leu Ile Phe Ala Asp Asp Leu Gly
            20                  25                  30

Tyr Gly Asp Leu Gly Cys Tyr Gly His Pro Ser Ser Thr Thr Pro Asn
        35                  40                  45

Leu Asp Gln Leu Ala Ala Gly Gly Leu Arg Phe Thr Asp Phe Tyr Val
    50                  55                  60

Pro Val Ser Leu Cys Thr Pro Ser Arg Ala Ala Leu Leu Thr Gly Arg

65                  70                  75                  80

Leu Pro Val Arg Met Gly Met Tyr Pro Gly Val Leu Val Pro Ser Ser
                85                  90                  95

Arg Gly Gly Leu Pro Leu Glu Glu Val Thr Val Ala Glu Val Leu Ala
            100                 105                 110

Ala Arg Gly Tyr Leu Thr Gly Met Ala Gly Lys Trp His Leu Gly Val
        115                 120                 125

Gly Pro Glu Gly Ala Phe Leu Pro Pro His Gln Gly Phe His Arg Phe
    130                 135                 140

Leu Gly Ile Pro Tyr Ser His Asp Gln Gly Pro Cys Gln Asn Leu Thr
145                 150                 155                 160

Cys Phe Pro Pro Ala Thr Pro Cys Asp Gly Gly Cys Asp Gln Gly Leu
                165                 170                 175

Val Pro Ile Pro Leu Leu Ala Asn Leu Ser Val Glu Ala Gln Pro Pro
            180                 185                 190

Trp Leu Pro Gly Leu Glu Ala Arg Tyr Val Ala Phe Ala His Asp Leu
        195                 200                 205

Met Ala Asp Ala Gln Arg Gln Asp Arg Pro Phe Phe Leu Tyr Tyr Ala
    210                 215                 220

Ser His His Thr His Tyr Pro Gln Phe Ser Gly Gln Ser Phe Ala Glu
225                 230                 235                 240

Arg Ser Gly Arg Gly Pro Phe Gly Asp Ser Leu Met Glu Leu Asp Ala
                245                 250                 255

Ala Val Gly Thr Leu Met Thr Ala Ile Gly Asp Leu Gly Leu Leu Glu
            260                 265                 270

Glu Thr Leu Val Ile Phe Thr Ala Asp Asn Gly Pro Glu Leu Met Arg
        275                 280                 285

Met Ser Asn Gly Gly Cys Ser Gly Leu Leu Arg Cys Gly Lys Gly Thr
    290                 295                 300

Thr Tyr Glu Gly Gly Val Arg Glu Pro Ala Leu Ala Phe Trp Pro Gly
305                 310                 315                 320

His Ile Ala Pro Gly Val Thr His Glu Leu Ala Ser Ser Leu Asp Leu
                325                 330                 335

Leu Pro Thr Leu Ala Ala Leu Ala Gly Ala Pro Leu Pro Asn Val Thr
            340                 345                 350

Leu Asp Gly Phe Asp Leu Ser Pro Leu Leu Leu Gly Thr Gly Lys Ser
        355                 360                 365

Pro Arg Gln Ser Leu Phe Phe Tyr Pro Ser Tyr Pro Asp Glu Val Arg
    370                 375                 380

Gly Val Phe Ala Val Arg Thr Gly Lys Tyr Lys Ala His Phe Phe Thr
385                 390                 395                 400

Gln Gly Ser Ala His Ser Asp Thr Thr Ala Asp Pro Ala Cys His Ala
                405                 410                 415

Ser Ser Ser Leu Thr Ala His Xaa Pro Pro Leu Leu Tyr Asp Leu Ser
            420                 425                 430

Lys Asp Pro Gly Glu Asn Tyr Asn Leu Leu Gly Gly Val Ala Gly Ala
        435                 440                 445

Thr Pro Glu Val Leu Gln Ala Leu Lys Gln Leu Gln Leu Leu Lys Ala
    450                 455                 460

Gln Leu Asp Ala Ala Val Thr Phe Gly Pro Ser Gln Val Ala Arg Gly
465                 470                 475                 480

Glu Asp Pro Ala Leu Gln Ile Cys Cys His Pro Gly Cys Thr Pro Arg
                485                 490                 495

Pro Ala Cys Cys His Cys Pro Asp Pro His Ala
            500                 505

<210>  5
<211>  538
<212>  PRT
<213>  artificial

<220>
<223>  mutated human ARSA with increased enzymatic activity and
       stability and apoEII

<220>
<221>  misc_feature
<222>  (424)..(424)
<223>  Xaa can be any naturally occurring amino acid

<400>  5

Met Gly Ala Pro Arg Ser Leu Leu Leu Ala Leu Ala Ala Gly Leu Ala
1               5                   10                  15

Val Ala Arg Pro Pro Asn Ile Val Leu Ile Phe Ala Asp Asp Leu Gly
            20                  25                  30

Tyr Gly Asp Leu Gly Cys Tyr Gly His Pro Ser Ser Thr Thr Pro Asn
        35                  40                  45

Leu Asp Gln Leu Ala Ala Gly Gly Leu Arg Phe Thr Asp Phe Tyr Val
    50                  55                  60

Pro Val Ser Leu Cys Thr Pro Ser Arg Ala Ala Leu Leu Thr Gly Arg
65                  70                  75                  80

Leu Pro Val Arg Met Gly Met Tyr Pro Gly Val Leu Val Pro Ser Ser
                85                  90                  95

Arg Gly Gly Leu Pro Leu Glu Glu Val Thr Val Ala Glu Val Leu Ala
            100                 105                 110

Ala Arg Gly Tyr Leu Thr Gly Met Ala Gly Lys Trp His Leu Gly Val
        115                 120                 125

Gly Pro Glu Gly Ala Phe Leu Pro Pro His Gln Gly Phe His Arg Phe
    130                 135                 140

Leu Gly Ile Pro Tyr Ser His Asp Gln Gly Pro Cys Gln Asn Leu Thr
145                 150                 155                 160

Cys Phe Pro Pro Ala Thr Pro Cys Asp Gly Gly Cys Asp Gln Gly Leu
                165                 170                 175

Val Pro Ile Pro Leu Leu Ala Asn Leu Ser Val Glu Ala Gln Pro Pro
            180                 185                 190

Trp Leu Pro Gly Leu Glu Ala Arg Tyr Val Ala Phe Ala His Asp Leu
        195                 200                 205

Met Ala Asp Ala Gln Arg Gln Asp Arg Pro Phe Phe Leu Tyr Tyr Ala
    210                 215                 220

Ser His His Thr His Tyr Pro Gln Phe Ser Gly Gln Ser Phe Ala Glu
225                 230                 235                 240

Arg Ser Gly Arg Gly Pro Phe Gly Asp Ser Leu Met Glu Leu Asp Ala

                245                 250                 255

Ala Val Gly Thr Leu Met Thr Ala Ile Gly Asp Leu Gly Leu Leu Glu
            260                 265                 270

Glu Thr Leu Val Ile Phe Thr Ala Asp Asn Gly Pro Glu Leu Met Arg
        275                 280                 285

Met Ser Asn Gly Gly Cys Ser Gly Leu Leu Arg Cys Gly Lys Gly Thr
    290                 295                 300

Thr Tyr Glu Gly Gly Val Arg Glu Pro Ala Leu Ala Phe Trp Pro Gly
305                 310                 315                 320

His Ile Ala Pro Gly Val Thr His Glu Leu Ala Ser Ser Leu Asp Leu
                325                 330                 335

Leu Pro Thr Leu Ala Ala Leu Ala Gly Ala Pro Leu Pro Asn Val Thr
            340                 345                 350

Leu Asp Gly Phe Asp Leu Ser Pro Leu Leu Leu Gly Thr Gly Lys Ser
        355                 360                 365

Pro Arg Gln Ser Leu Phe Phe Tyr Pro Ser Tyr Pro Asp Glu Val Arg
    370                 375                 380

Gly Val Phe Ala Val Arg Thr Gly Lys Tyr Lys Ala His Phe Phe Thr
385                 390                 395                 400

Gln Gly Ser Ala His Ser Asp Thr Thr Ala Asp Pro Ala Cys His Ala
                405                 410                 415

Ser Ser Ser Leu Thr Ala His Xaa Pro Pro Leu Leu Tyr Asp Leu Ser
            420                 425                 430

Lys Asp Pro Gly Glu Asn Tyr Asn Leu Leu Gly Gly Val Ala Gly Ala
        435                 440                 445

Thr Pro Glu Val Leu Gln Ala Leu Lys Gln Leu Gln Leu Leu Lys Ala
    450                 455                 460

Gln Leu Asp Ala Ala Val Thr Phe Gly Pro Ser Gln Val Ala Arg Gly
465                 470                 475                 480

Glu Asp Pro Ala Leu Gln Ile Cys Cys His Pro Gly Cys Thr Pro Arg
                485                 490                 495

Pro Ala Cys Cys His Cys Pro Asp Pro His Ala Ser Ser Ala Trp Ser
            500                 505                 510

His Pro Gln Phe Glu Lys Gly Gly Gly Ser Gly Gly Gly Ser Gly Gly
        515                 520                 525

Ser Ala Trp Ser His Pro Gln Phe Glu Lys
    530                 535

<210>  6
<211>  30
<212>  PRT
<213>  artificial

<220>
<223>  apoEII tag

<400>  6

Ser Ala Trp Ser His Pro Gln Phe Glu Lys Gly Gly Gly Ser Gly Gly
1               5                   10                  15

Gly Ser Gly Gly Ser Ala Trp Ser His Pro Gln Phe Glu Lys
            20                  25                  30

<210>  7
<211>  10
<212>  PRT
<213>  artificial

<220>
<223>  apoEII binding domain

<400>  7

Ser Ala Trp Ser His Pro Gln Phe Glu Lys
1               5                   10

<210>  8
<211>  10
<212>  PRT
<213>  artificial

<220>
<223>  linker

<400>  8

Gly Gly Gly Ser Gly Gly Gly Ser Gly Gly
1               5                   10

## Description

### FIELD OF THE INVENTION

The invention is based on the introduction of mutations into the amino acid sequence of human Arylsulfatase A (ASA or ARSA) in order to increase protein stability. The invention introduces amino acid mutations, such as deletions, substitutions or additions, into the C-terminal part of the human ARSA enzyme, in particular at a position around or at amino acid 424, which result in a sequence that does not comprise E424. Provided are further nucleic acids and vectors for the expression of the mutated ARSA of the invention, recombinant cells and pharmaceutical composition comprising the mutated ARSA, as well as its use in the treatment of diseases that are characterized by a reduced activity of endogenous ARSA.

### DESCRIPTION

Metachromatic leukodystrophy (MLD) (from the Greek word leukos for "white", dys for "lack of', and troph for "growth") is an autosomal recessive lysosomal disorder caused by the deficiency in the enzymatic activity of arylsulfatase A (ARSA or ASA, EC 3.1.6.8), re-sulting in impaired degradation of 3-O-sulfogalactosylceramide (sulfatide), an essential sphingolipid of myelin (Gieselmann V & Krägeloh-Mann I, Neuropediatrics. 2010, 41, 1-6; Eckhardt M, Mol Neurobiol. 2008, 37: 93-103.). ARSA hydrolyzes sulfatide to galacto-sylceramide and sulfate and is, due to the lack of alternative degradation pathways, indispensable for sulfatide recycling. Impairment of ARSA function results in increased accumulation of sulfatide which clinically manifests in progressive demyelination and neurological symptoms resulting in severe debilitation and eventually death of the affected patient. MLD is a rare disorder with a prevalence ranging from 1:40000 to 1:100000. The deficiency in the ARSA enzyme is caused by mutations in the ARSA gene in homo- or compound heterozygosity. Many mutations in the ARSA gene have been identified to date, more than 200 of which are known to cause the deleterious MLD disease. MLD can manifest itself in young children (late infantile form), where affected children typically begin showing symptoms just after the first year of life (e.g., at about 15-24 months), and death usually occurs about 5 years after onset of clinical symptoms. MLD can manifest itself in children (juvenile form), where affected children typically show cognitive impairment by about the age of 3-10 years, and lifespan can vary (e.g., in the range of 10-15 years after onset of symptoms). MLD can manifest itself in adults at various ages beyond puberty (age 16 and later). The progression of such adult-onset forms can vary greatly.

ARSA has been purified from a variety of sources including human liver, placenta, and urine. It is an acidic glycoprotein with a low isoelectric point. It has a molecular mass of approximately 60 kDa. Above pH 6.5, the enzyme exists as a homodimer. ARSA undergoes a pH-dependent polymerisation and forms octamers below pH 5.6. In human urine, the enzyme consists of two non-identical subunits of 63 and 54 kDa. ARSA purified from human liver, placenta, and fibroblasts also consists of two subunits of slightly different sizes varying between 55 and 64 kDa. As in the case of other lysosomal enzymes, ARSA is synthesised by membrane-bound ribosomes as a glycosylated precursor. It then passes through the endoplasmic reticulum and Golgi, where its N-linked oligosaccharides are processed with the formation of phosphorylated mannosyl residues that are required for lysosomal targeting via mannose 6-phosphate receptor binding (Sommerlade et al., J Biol Chem. 1994, 269: 20977-81; Coutinho MF et al., Mol genet metabol. 2012, 105: 542-550).

An unusual protein modification is essential for the enzymatic activity of all 17 human sulfatases known to date. It has been initially identified in ARSA, arylsulfatase B (ARSB) and a sulfatase from the green alga Volvox carteri (Schmidt B et al. Cell. 1995, 82, 271-278, Selmer T et al. Eur J Biochem. 1996, 238, 341-345). This modification leads to the conversion of an active site cysteine residue, which is conserved among the known sulfatases, into a 2-amino-3-oxopropionic acid residue also termed Cα-formylglycine (FGly) (Schmidt B et al. Cell. 1995, 82, 271-278). The formylglycine-generating enzyme (FGE) catalyzes this conversion. A lack of FGE activity causes a combined functional deficiency of all human sulfatases, a severe lysosomal storage disease called multiple sulfatase deficiency (MSD). In ARSA and ARSB the conversion of the Cys-69 and Cys-91 residue, respectively, to FGly is required for generating a catalytically active enzyme. Cys-69 is referred to the precursor ARSA which has an 18 residue signal peptide. In the mature ARSA the mentioned cysteine residue is Cys-51. Further investigations have shown that a linear sequence of 16 residues surrounding the Cys-51 in the mature ARSA is sufficient to direct the conversion and that the protein modification occurs after or at a late stage of co-translational protein translocation into the endoplasmic reticulum when the polypeptide is not yet folded to its native structure (Dierks T et al. Proc Natl Acad Sci. 1997, 94, 11963-1196).

Since MLD is caused by defective ARSA, most therapeutic approaches have tried to correct the biochemical defect by providing wild-type ARSA. The different methods and sources of wild-type ARSA constitute distinct therapeutic approaches (Sevin et al., J Inherit Metab Dis. 2007, 30, 175-83; Shaimardanova et al., Front Med (Lausanne) 2020, 7, 576221). Hematopoietic stem cell transplantation (HSCT) is the transplantation of hematopoietic stem cells from a healthy donor. After engraftment, progenies of donor-derived cells differentiate into the different cell types of the hematopoeitic system and provide wild-type ARSA to patient's cells via release-recapture pathways. These pathways are based on the pecularities of the sorting process of newly synthesized soluble lysosomal enzymes which may involve partial secretion of newly synthesized lysosomal enzymes and subsequent uptake by neighbouring cells expressing mannose 6-phosphate receptors. Cellular uptake may also be accomplished by mannose receptors, asialoglycoprotein receptors, scavenger receptors or other endocytic receptors on the cell surface that can bind exogenous lysosomal enzymes. In addition, receptor-independent uptake via direct cell-cell contacts or tunnelling nanotubes have been described. Many MLD patients have been treated by allogeneic HSCT with varying success. Enzyme replacement therapy (ERT) relies on providing recombinantly expressed wild-type human ARSA to patients. Repeated intravenous injection of therapeutic enzyme proved to be effective in a number of lysosomal storage diseases and is clinically approved for eight of them. For MLD, two clinical trials using either intravenous or intrathecal infusion of recombinant ARSA have been launched (see below). Also gene therapy approaches are presently in the clinical evaluation. They are generally based on the overexpression of wild-type ARSA in patient's own cells by transducing them with appropriate expression vectors. This can be done either by injecting appropriate expression vectors directly into the tissue (in vivo gene therapy) or by transducing patient's cells outside the body (ex vivo gene therapy). Also in this treatment regimen the overexpressing cells may serve as an enzyme source for deficient cells. An ex vivo gene therapy approach using lentiviral gene transfer to overexpress ARSA in autologous CD34+ hematopoietic stem cells is in a phase 1/2 clinical trial (see https://clinicaltrials.gov/ct2/show/NCT01560182). The approach was successful in a mouse model of MLD (Biffi A, et al., J Clin Invest. 2004, 113: 1118-29.). In another gene therapy trial an adenovirus-associated vector encoding wild-type human ARSA has been injected directly into the brain of children affected with early onset forms of MLD (see https://clinicaltrials.gov/ct2/show/NCT01801709). Also this in vivo gene therapy approach has demonstrated therapeutic benefit in a mouse model of MLD (Piguet F et al. Hum Gene Ther. 2012, 23, 903-14). Results from the clinical trial have not been reported. Other cell based gene therapies for replacing ARSA try to use microencapsulated recombinant cells, oligodendrocyte progenitor cells, and neural progenitor cells as well as embryonic stem cells. All treatment approaches have limitations and bear certain risks. Also producing the enzyme for ERT in high purity and in large scale recombinantly has been a problem. Recently a study of ERT with wild-type ARSA has shown limited efficacy in MLD (i Dali et al., 2016, Mol Gen Metabol. 117, 73). Three cohorts of 6 patients each were treated with 10, 30 or 100 mg of wild-type ARSA every two weeks in a total of 40 weeks treatment schedule. To circumvent the blood-brain barrier, the enzyme was administered into the cerebrospinal fluid via intrathecal injections. Only few immunological adverse effects were observed. Although this phase 1/2 clinical trial did not involve a placebo-treated control group, conclusions can be drawn by comparing the different dose groups. Importantly, the group treated with 100 mg showed a significantly reduced deterioration of motor functions compared to the group treated with 10 mg. However, treatment effectivity still suffers from targeting sufficient enzyme activity to the central nervous system. This is particularily problematic if intravenous injection is used to provide enzyme to the patient as the blood-brain barrier prevents efficient transfer of ARSA from the blood circulation to the brain parenchyma. Preclinical studies in mouse models of MLD had shown that weekly ARSA doses of at least 20 mg per kg body weight are required to improve sulfatide storage in the brain (Matzner et al., Mol Ther, 2009, 17, 600-606). The requirement of high doses in mice explains the failure of a recent clinical trial testing repeated intravenous injection of up to 5 mg/kg ARSA in early-onset MLD (see https://clinicaltrials.gov/ct2/show/results/NCT00418561; Dali et al., 2021, Ann Clin Transl Neurol. 8:66-80.). The enzyme activity accumulating in the brain might have been below the threshold required for therapeutic effects. Increasing the ARSA-doses is not a preferred solution to increase enzyme levels in the brain and treatment effectivity. Higher enzyme doses are more likely to induce the generation of neutralizing antibodies directed to the expressed protein which might result in severe adverse effects including anaphylaxis. Therefore, there is a need to increase treatment effectivity of ARSA enzyme replacement. The same holds true for gene therapy because a relatively small number of producer cells has to supply ARSA activity to a large number of ARSA-deficient brain cells. In such approaches, an excessive expression of wild-type ARSA might have adverse effects because the overexpressed enzyme can saturate FGE in the endoplasmic reticulum of the producer cells and cause an inefficient post-translational activation of ARSA and other cellular sulfatases. Also the cellular machinery generating mannose 6-phosphate residues might be overloaded, resulting in the delivery of uptake-incompetent enzyme.

WO 2018/141958 discloses a mutated ARSA enzyme having amino acid variations at position 202, 286 and/or 291 of wild-type human ARSA. The disclosed mutated ARSA enzymes show an up to 5-fold increased enzymatic activity (see also Simonis, et al. (2019) Hum Mol Genet. 28:1810).

ARSA is known as a target for several lysosomal thiol proteases which have a significant impact on enzyme half-life. Increases of the protein stability and enzyme half-life by genetic engineering could be another route to improve protein replacement treatments in diseases such as Metachromatic leukodystrophy. Hence, stabilization of ARSA was an object of the present invention to provide further improved mutated ARSA enzymes as a therapeutic compound for the treatment of diseases associated with a pathogenic decreased endogenous enzymatic activity of ARSA.

### BRIEF DESCRIPTION OF THE INVENTION

Generally, and by way of brief description, the main aspects solving the above problem of the present invention can be described as follows:
In **a first aspect,** the invention pertains to a mutated arylsulfatase A (ARSA) enzyme, comprising an amino acid sequence with at least 80%, 85%, 90%, 95%, 96%, 97%, most preferably at least 99% sequence identity to SEQ ID NO: 1 (human ARSA enzyme), wherein the mutated ARSA enzyme amino acid sequence when aligned to the sequence of SEQ ID NO: 1, comprises at least one mutation compared to the sequence between residues 350 and 450 of SEQ ID NO: 1.
In **a second aspect,** the invention pertains to an isolated nucleic acid or vector construct encoding the mutated ARSA of the invention of the first aspect.
In **a third aspect,** the invention pertains to a recombinant cell comprising the mutated ARSA enzyme according to the first aspect, a nucleic acid or a vector according to the second aspect.
In **a fourth aspect,** the invention pertains to a pharmaceutical composition comprising the mutated ARSA of the invention, or a nucleic acid, vector or cell of the invention.
In **a fifth aspect,** the invention pertains to a use of the compounds of the invention in the treatment of a disease, such as preferably a genetic disease characterized by a pathological insufficiency of endogenous ARSA, such as it is the case in metachromatic leukodystrophy.
In **an alternative aspect,** the invention provides a method for treating a disease in a subject comprising administering to the subject a therapeutically effective amount of a compound of the invention.
In **a sixth aspect,** the invention further provides a method for producing a mutated ARSA of the invention, preferably wherein the method comprises a step of recombinantly expressing the mutated ARSA protein.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a mutated arylsulfatase A (ARSA) enzyme, comprising an amino acid sequence with at least 80%, 85%, 90%, 95%, 96%, 97%, most preferably at least 99% sequence identity to SEQ ID NO: 1 (human ARSA enzyme), wherein the mutated ARSA enzyme amino acid sequence when aligned to the sequence of SEQ ID NO: 1, comprises at least one mutation compared to the sequence between residues 350 and 450 of SEQ ID NO: 1.

In one preferred embodiment, the mutated ARSA of the invention is a protein having an increased protein stability compared to the enzyme of SEQ ID NO: 1. In the context of the herein disclosed invention the term "protein stability" or "stability" is generally used in a structural context, i.e. relating to the structural integrity and half-life of a protein, or in a functional context, i.e. relating to a protein's ability to retain its function and/or activity over time. Protein stability can be influenced by proteolytic cleavage, loss of structural integrity of the three-dimensional folding, general physiological protein turn-over. Protein stability can be measured by a wide range of processes known to the skilled artisan and include, without being limited to any particular method, immunological methods using antibodies binding to three dimensional epitopes, pulse-chase methods such as cyclohexamide chase and functional assays measuring time-dependent decline of enzyme activity

As used herein, the terms "identical" or percent "identity", when used anywhere herein in the context of two or more nucleic acid or protein/polypeptide sequences, refer to two or more sequences or subsequences that are the same or have (or have at least) a specified per-centage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or desig-nated region) as measured using a sequence comparison algorithms, or by manual alignment and visual inspection (see, e.g., NCBI web site). In a particular embodiment, for example when comparing the protein or nucleic acid sequence of a mutated ARSA with wild-type ARSA, the percentage identity can be determined by the Blast searches or local alignments; in particular for amino acid identity, those using BLASTP 2.2.28+ with the following pa-rameters: Matrix: BLOSUM62; Gap Penalties: Existence: 11, Extension: 1; Neighboring words threshold: 11; Window for multiple hits: 40.

The term "mutation" refers to, in the context of a polynucleotide, a modification to the polynucleotide sequence resulting in a change in the sequence of a polynucleotide with reference to a precursor polynucleotide sequence. A mutant polynucleotide sequence can refer to an alteration that does not change the encoded amino acid sequence, for example, with regard to codon optimization for expression purposes, or that modifies a codon in such a way as to result in a modification of the encoded amino acid sequence - such "silent" mutations are however in context of the present invention in preferred embodiments excluded. Mutations can be introduced into a polynucleotide through any number of methods known to those of ordinary skill in the art, including random mutagenesis, site-specific mutagenesis, oligonucleotide directed mutagenesis, gene shuffling, directed evolution techniques, combinatorial mutagenesis, site saturation mutagenesis among others.

"Mutation" or "mutated" means, in the context of a protein, a modification to the amino acid sequence resulting in a change in the sequence of a protein with reference to a precursor protein sequence. A mutation can refer to a substitution of one amino acid with another amino acid, an insertion or a deletion of one or more amino acid residues. Specifically, a mutation can also be the replacement of an amino acid with a non-natural amino acid, or with a chemically-modified amino acid or like residues. A mutation can also be a truncation (e.g., a deletion or interruption) in a sequence or a subsequence from the precursor sequence. In context of the present invention a mutation is preferably an alteration that results into a removal of a certain amino acid side chain targeted by the mutation. A mutation may also be an addition of a subsequence (e.g., two or more amino acids in a stretch, which are inserted between two contiguous amino acids in a precursor protein sequence) within a protein, or at either terminal end of a protein, thereby increasing the length of (or elongating) the protein. A mutation can be made by modifying the DNA sequence corresponding to the precursor protein. Mutations can be introduced into a protein sequence by known methods in the art, for example, by creating synthetic DNA sequences that encode the mutation with reference to precursor proteins, or chemically altering the protein itself. A "mutant" as used herein is a protein comprising a mutation. For example, it is also possible to make a mutant by replacing a portion of ARSA with a wild-type sequence of human or non-human origin (preferably non-human vertebrates) that corresponds to such portion but includes a desired variation at a specific position that is naturally-occurring in the wild-type sequence.

The use of the mutated ARSA enzymes, or of the functional fragment thereof, of the present invention overcomes the problems in the art because the provided mutations increased protein stability (and activity over time to metabolize sulfatides) which allows to maintain low enzyme concentrations/expressions while increasing enzyme activity over time. Also, problems of expressing sufficient amount of enzyme activity either recombinant (ERT) or in situ (gene therapy) is overcome by the herein provided highly "super stable" ARSA variant. The mutated ARSA of the invention shows a 2-fold to more than 28-fold increased protein half-life compared to the human wild-type enzyme.

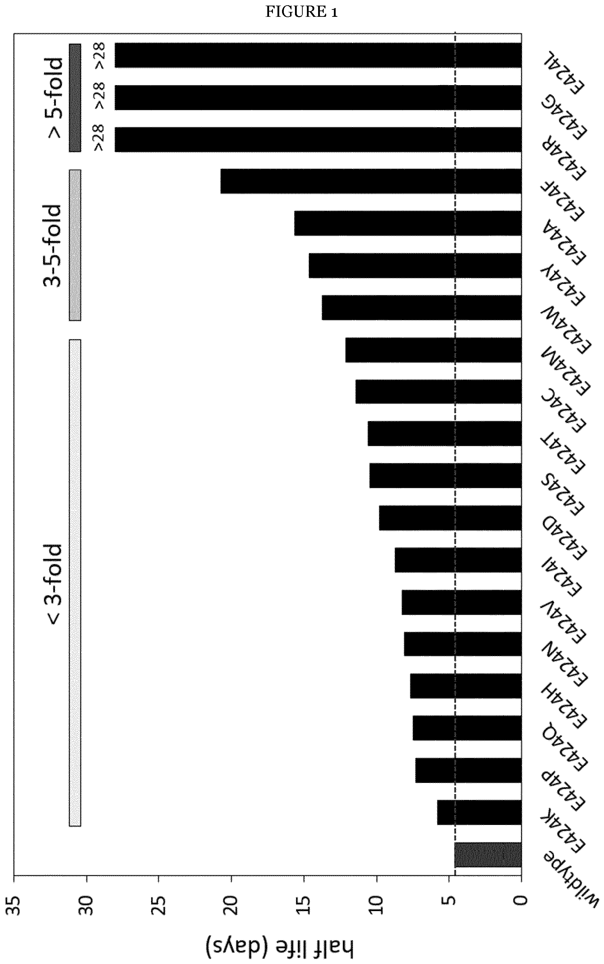

In preferred embodiments of the invention the at least one mutation is between amino acids 420 and 430, preferably between 422 and 428, of SEQ ID NO: 1. Even more preferably, the at least one mutation is at amino acid position 424 of SEQ ID NO: 1, and preferably is a mutation, such as a deletion or substitution of amino acid E424. Surprisingly, the present invention shows that a change of the E424 amino acid into any other amino acid results into an increase of protein half-life (see Figure 1). Accordingly, the present invention provides evidence that 19 variations of mutations result in the same effect, and therefore, the invention in preferred embodiments pertains to a mutated human ARSA having a mutation at position 424 that results in a loss of the wild-type glutamic acid amino acid side chain at position 424 of SEQ ID NO: 1, which can be either a modification of the side chain, a substitution or a deletion.

However, some preferred embodiments may pertain to a mutation into any other AA, a substitution with an W, Y, A, F, R, G, L and in some specific embodiments is preferably is E424R, E424G or E424L.

A preferred mutated ARSA in accordance with the herein disclosed invention comprises, or consists essentially of, the amino acid sequence shown in SEQ ID NO: 4 or 5.

In certain embodiments of the invention it is preferred that the mutated ARSA of the invention, which shows increased protein stability / half-life further comprises additional modifications that are known as beneficial for, for example, enzyme activity. In particular included in the present disclosure are therefore modifications as disclosed in WO 2018/141958 (incorporated herein by reference in its entirety). Hence, in accordance with the invention, preferably, a mutated ARSA of the invention may include at least one, preferably at least two or three, additional mutations, which in certain embodiments are murinized amino acid changes in the sequence of SEQ ID NO:1, in other words, constitute a change which is a replacement of a human amino acid with the corresponding amino acid in the murine ARSA sequence. Specifically preferred therefore, are mutated ARSA enzymes wherein the mutated ARSA enzyme amino acid sequence when aligned to the sequence of SEQ ID NO: 1, further comprises at least one, in specific embodiments at least two and most preferably at least three, further mutation compared to the sequence between residues 150 and 350 of SEQ ID NO: 1. For example, the mutated ARSA enzyme amino acid sequence when aligned to the sequence of SEQ ID NO: 1, may further comprises at least one further mutation, in specific embodiments at least two and most preferably at least three mutations, compared to the sequence between residues 180 to 220, and/or 260 to 320 of SEQ ID NO: 1. Such additional mutations are preferably selected from a mutation, which when compared to the sequence of SEQ ID NO: 1, is located between residues 195 to 210, and/or 280 to 300, and most preferably is a mutation at a position selected from amino acid positions 202, 286 and/or 291 of SEQ ID NO: 1. Most preferably, the mutated ARSA enzyme amino acid sequence when aligned to the sequence of SEQ ID NO: 1, comprises at least one further mutation selected from M202V, T286R and/or R291N compared to SEQ ID NO: 1, preferably of at least M202V. Most preferably, the mutated ARSA of the invention comprises at least two further mutations, preferably all three, selected from M202V, T286R and R291N when compared to SEQ ID NO: 1.

A further surprising result of the present invention is that, and therefore this result constitutes a preferred embodiment, the mutated ARSA enzyme retains an enzymatic activity of degradation of sulfatides, preferably an activity of degradation of cerebroside 3-sulfate into cerebroside and sulfate. Even more preferably, the mutated ARSA enzyme has an increased activity compared to human wild-type ARSA, for example by introducing one or more mutations disclosed in WO 2018/141958, or herein above.

The present invention is predicated upon the fact that an amino acid change at the C-terminal region of human ARSA, in particular the sections as defined herein above, result in increased protein stability and half-life, and therefore, the invention pertains in preferred embodiments to a mutated ARSA characterized by an increased protein half-life compared to a wild-type human ARSA of SEQ ID NO: 1; and/or has a decreased mannose 6-phosphorylation of lysosomal proteins compared to a wild-type human ARSA of SEQ ID NO: 1. Such increased protein half-life may be determined according to a method and under the conditions as shown in the examples below.

Further preferred embodiments pertain to a mutated ARSA of the invention which comprises further N- or C-terminal tags. A preferred tag is a C-terminally attached apoE-II protein which is an apolipoprotein E (apoE)-derived peptide sequence comprising, preferably consisting of, a tandem repeat of its low-density lipoprotein receptor binding domain (such as SEQ ID NO: 7). A preferred apoEII tag according to the invention is shown in SEQ ID NO: 6, or at least comprises one or more repeats of the sequence shown in SEQ ID NO: 7, optionally connected by a linker (SEQ ID NO: 8).

The mutated ARSA enzyme according to any one of claims 1 to 15, comprising compared to SEQ ID NO: 1 the mutations at positions M202, T286, R291 and E424, and a C-terminal covalently attached apoEII protein sequence. ApoE is the only serum apolipoprotein that is also found in the extravascular fluid of the brain, and its receptor binding domain - tandem dimer repeat peptide - induces endocytosis by neurons and other cells via a receptor associated protein sensitive pathway (Wang X, et al. Brain Res. 1997;778:6-15. doi: 10.1016/S0006-8993(97)00877-9). Such an apo E II tag is preferably attached to the C-terminus of the mutated ARSA of the invention in order to increase transcytosis across the blood-brain barrier (Böckenhoff et al., J Neurosci. 2014, 34, 3122-3129) and to increase endocytosis by neurons, astrocytes and oligodendrocytes.

The mutated ARSA enzyme, or the functional fragment thereof, of the invention in preferred embodiments retains an enzymatic activity of degradation of sulfatides, preferably an activity of degradation of 3-O-sulfogalactosylceramide into galactosylceramide and sulfate. Preferably the mutated ARSA enzyme, or the functional fragment thereof, of the invention has an increased aforementioned activity compared to human wild-type ARSA.

The mutated ARSA of the invention is in preferred embodiments an isolated ARSA or a recombinant ARSA polypeptide. The term "recombinant" or "recombinantly produced" in context of the invention means that a protein or peptide is expressed via an artificially intro-duced exogenous nucleic acid sequence in a biological cell. Recombinant expression is usu-ally performed by using expression vectors as described herein elsewhere.

In a preferred embodiment of the invention the mutated ARSA enzyme can assemble as a protein octamer pH-independently already at neutral pH, therefore, the mutated ARSA enzyme of the invention is able to oligomerize into an ARSA protein particle comprising multiple ARSA protein monomers, preferably wherein the number ARSA monomers in the particle consists of eight ARSA protein monomers. It was surprisingly identified that the mutated ARSA of the invention exists as a protein octamer already at neutral pH, and, without being bound by theory, the octameric assembly results in a reduced mannose-6-phosphate (M6P) modification of the protein and therefore decreases ARSA liver dependent depletion. The mutated ARSA of the invention hence has a further advantage that the reduced M6P modification allows a redistribution of enzyme activity in favour of the central nervous system and an M6P independent trafficking of the protein to the lysosome.

In a **second aspect,** the invention pertains to an isolated nucleic acid or vector construct encoding the mutated ARSA of the invention of the first aspect.

In the second aspect the problem is solved by an isolated nucleic acid comprising a sequence coding for the mutated ARSA enzyme as described herein before, or encoding for a functional fragment of a mutated ARSA enzyme as described herein before. The term "encoding" or more simply "coding" refers to the ability of a nucleotide sequence to code for one or more amino acids. The term does not require a start or stop codon. An amino acid sequence can be encoded in any one of six different reading frames provided by a polynucleotide sequence and its complement. An amino acid sequence can be encoded by desoxyribonucleic acid (DNA), ribonucleic acid (RNA), or artificially synthesized polymers similar to DNA or RNA.

Another aspect of the invention provides a vector, comprising the nucleic acid of the invention. A "vector" may be any agent that is able to deliver or maintain a nucleic acid in a host cell and includes, for example, but is not limited to, plasmids (e.g., DNA plasmids), naked nucleic acids, viral vectors, viruses, nucleic acids complexed with one or more polypeptide or other molecules, as well as nucleic acids immobilized onto solid phase particles. Vectors are described in detail below. A vector can be useful as an agent for delivering or maintaining an exogenous gene and/or protein in a host cell. A vector may be capable of transducing, transfecting, or transforming a cell, thereby causing the cell to replicate or express nucleic acids and/or proteins other than those native to the cell or in a manner not native to the cell. The target cell may be a cell maintained under cell culture conditions or in other in vivo embodiments, being part of a living organism. A vector may include materials to aid in achieving entry of a nucleic acid into the cell, such as a viral particle, liposome, protein coating, or the like. Any method of transferring a nucleic acid into the cell may be used; unless other-wise indicated, the term vector does not imply any particular method of delivering a nucleic acid into a cell or imply that any particular cell type is the subject of transduction. The pre-sent invention is not limited to any specific vector for delivery or maintenance of any nucleic acid of the invention, including, e.g., a nucleic acid encoding a mutant ARSA polypeptide of the invention or a fragment thereof.

Preferably the vector of the invention is an expression vector. The term "expression vector" typically refers to a nucleic acid construct or sequence, generated recombinantly or synthetically, with a series of specific nucleic acid elements that permit transcription of a particular nucleic acid in a host cell. The expression vector typically includes a nucleic acid to be transcribed - the mutated ARSA of the invention - operably linked to a promoter. The term "expression" includes any step involved in the production of the polypeptide including, but not limited to, transcription, post-transcriptional modification, translation, post-translational modification, and/or secretion. A preferred vector of the invention is a plant-specific, bacterial, yeast, insect, vertebrate, preferably mammalian, or a viral vector, preferably retroviral and adeno-associated viral vector. Preferred vectors of the invention are suitable for use in gene therapy, preferably gene therapy based on transformation of autologous adult stem cells.

In **a third aspect,** the invention pertains to a recombinant cell comprising the mutated ARSA enzyme according to the first aspect, a nucleic acid or a vector according to the second aspect.

A "recombinant cell" or also referred to as "host cell" is any cell that is susceptible to transformation with a nucleic acid. Preferably the recombinant or host cell of the invention is a plant cell, bacterial cell, yeast cell, an insect cell or a vertebrate, preferably a mammalian, cell. A preferred recombinant cell is selected from a cell suitable for recombinant expression of the mutated ARSA of the invention. Most preferred is a Chinese hamster ovary (CHO) cell. Also preferred are human cells, preferably autologous human cells derived from patient suffering from a disease described herein that is treatable with a mutated ARSA of the invention. A preferred human cell is a hematopoietic stem cell (HSC).

In **a fourth aspect,** the invention pertains to a pharmaceutical composition comprising the mutated ARSA of the invention, or a nucleic acid, vector or cell of the invention.

In the following the mutated ARSA, nucleic acids encoding the same, vectors and cells comprising these nucleic acids or mutated proteins, as well as pharmaceutical compositions thereof, will be referred to generally as "compounds of the invention".

As used herein the language "pharmaceutically acceptable carrier" is intended to include any and all solvents, solubilizers, fillers, stabilizers, binders, absorbents, bases, buffering agents, lubricants, controlled release vehicles, diluents, emulsifying agents, humectants, lubricants, dispersion media, coatings, antibacterial or antifungal agents, isotonic and absorption delay-ing agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its in-tended route of administration. Examples of routes of administration include parenteral, e.g., intrathecal, intracerebroventricular, intraparenchymal, intra-arterial, intravenous, intradermal, subcutaneous, oral, transdermal (topical) and transmucosal administration. The term "intrathecal," as used herein, means introduced into or occurring in the space under the arachnoid membrane which covers the brain and spinal cord. The term "intracerebroventric-ular" refers to administration of a composition into the ventricular system of the brain, e.g., via injection, infusion, or implantation (for example, into a ventricle of the brain). As used herein, the term "intraparenchymal" can refer to an administration directly to brain tissue. In other instances, intraparenchymal administration may be directed to any brain region where delivery of one or more compounds of the invention is effective to mitigate or prevent one or more of disorders as described herein.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride, mannitol or dextrose. pH can be adjusted with acids or bases, such as hy-drochloric acid or sodium hydroxide. The parenteral preparation can be enclosed in am-poules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or disper-sion medium containing, for example, water, ethanol, polyol (for example, glycerol, manni-tol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a compound of the invention such as a mutated ARSA) in the required amount in an appropri-ate solvent with one or a combination of ingredients enumerated above, as required, fol-lowed by filtered sterilization. Generally, dispersions are prepared by incorporating the ac-tive compound of the invention into a sterile vehicle which contains a basic dispersion me-dium and the required other ingredients from those enumerated above. In the case of sterile powders for the preparation of sterile injectable solutions, the preferred methods of prepara-tion are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, cap-sules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or con-trolled release of the active ingredient. Biodegradable, biocompatible polymers can be used, such as ethylene vinyl acetate, polyanhydrides, polyglycolic acid, collagen, serum albumin, polyorthoesters, polylactic acid, poly(butyl cyanoacrylate), and poly(lactic-co-glycolic) acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the de-sired therapeutic effect in association with the required pharmaceutical carrier. The specifi-cation for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dos-age may vary within this range depending upon the dosage form employed and the route of ad-ministration utilized. For any compound used in the method of the invention, the therapeutically effective dose can be estimated initially from cell culture assays. A dose may be formulated in animal models to achieve a circulating plasma concentration range that in-cludes the IC50 (i.e., the concentration of the test compound which achieves a half-maximal inhibition of symptoms) as determined in cell culture. Such information can be used to more accurately determine useful doses in humans. The pharmaceutical compositions can be in-cluded in a container, pack, or dispenser together with instructions for administration.

In **a fifth aspect,** the invention pertains to a use of the compounds of the invention in the treatment of a disease, such as preferably a genetic disease characterized by a pathological insufficiency of endogenous ARSA, such as it is the case in metachromatic leukodystrophy.

In **an alternative aspect,** the invention provides a method for treating a disease in a subject comprising administering to the subject a therapeutically effective amount of a compound of the invention.

The problem is furthermore solved in this fifth and alternative aspect by a medical use of the compounds of the invention in the treatment of a disease. The disease is preferably a disease characterized by a pathological enzymatic insufficiency of endogenous (human) ARSA. Generally preferred diseases are demyelinating disorders. In other preferred embodiments the disease is a leukodystrophy. A leukodystrophy in context with the present invention is preferably selected from metachromatic leukodystrophy, multiple sulfatase deficiency, Krabbe disease, adrenoleukodystrophy, Pelizaeus-Merzbacher disease, Canavan disease, Childhood Ataxia with Central Hypomyelination or CACH (also known as Vanishing White Matter Disease), Alexander disease, Refsum disease, and cerebrotendinous xanthomatosis. In most preferred embodiments of the invention the disease is metachromatic leukodystrophy (MLD).

Compositions and methods of the present invention may be used to effectively treat individuals (patients or subjects) suffering from or susceptible to MLD. The terms, "treat" or "treatment", as used herein, refers to amelioration of one or more symptoms associated with the disease, prevention or delay of the onset of one or more symptoms of the disease, and/or lessening of the severity or frequency of one or more symptoms of the disease. Exemplary symptoms include, but are not limited to, intracranial pressure, hydrocephalus ex vacuo, accumulated sulfated glycoli-pids in the myelin sheaths in the central and peripheral nervous system and in visceral organs, progressive demyelination and axonal loss within the CNS and PNS, and/or motor and cognitive dysfunction, like gait disturbances, mental regression, ataxia, loss of speech, spastic tetraparesis, or optic atrophy.

In some embodiments, treatment refers to partially or complete alleviation, amelioration, relief, inhibition, delaying onset, reducing severity and/or incidence of neurological impairment in an MLD patient. As used herein, the term "neurological impairment" includes various symptoms associated with impairment of the central nervous system (brain and spinal cord). In some embodiments, various symptoms of MLD are associated with impairment of the peripheral nervous system (PNS). In some embodiments, neurological impairment in an MLD patient is characterized by decline in gross motor function. It will be appreciated that gross motor function may be assessed by any appropriate method known to the skilled artisan.

In some embodiments, treatment refers to decreased sulfatide accumulation in various tis-sues. In some embodiments, treatment refers to decreased sulfatide accumulation in brain target tissues, spinal cord neurons, and/or peripheral target tissues. In certain embodiments, sulfatide accumulation is decreased by about 5%, 10%, 15%, 20%, 25%, 30%, 35%, 40%, 45%, 50%, 55%, 60%, 65%, 70%, 75%, 80%, 85%, 90%, 95%, 100% or more as compared to a control. In some embodiments, sulfatide accumulation is decreased by at least 1- fold, 2-fold, 3-fold, 4-fold, 5-fold, 6-fold, 7-fold, 8-fold, 9-fold or 10-fold as compared to a control. It will be appreciated that sulfatide storage may be assessed by any appropriate method. For example, in some embodiments, sulfatide storage is measured by alcian blue staining. In some embodiments, sulfatide storage is measured by high-performance liquid chromatography, thin layer chromatography or mass spectrometry.

In some embodiments, treatment refers to reduced vacuolization or a reduced number and/or size of alcian blue-positive storage deposits in neurons (e.g. in nuclei of the medulla oblon-gata and pons, and in several nuclei of midbrain and forebrain), astrocytes, oligoden-droctes, Schwann cells and/or microglial cells. In certain embodiments, vacuolization or storage deposits in these cell types are decreased by about 5%, 10%, 15%, 20%, 25%, 30%, 35%, 40%, 45%, 50%, 55%, 60%, 65%, 70%, 75%, 80%, 85%, 90%, 95%, 100% or more as compared to a control. In some embodiments, vacuolization or storage deposits are de-creased by at least 1-fold, 2-fold, 3-fold, 4-fold, 5-fold, 6-fold, 7- fold, 8-fold, 9-fold, 10-fold or more as compared to a control.

In some embodiments, treatment refers to increased ARSA enzyme activity in various tissues. In some embodiments, treatment refers to increased ARSA enzyme activity in brain target tissues, spinal cord, peripheral nerves and/or other peripheral target tissues. ARSA enzyme activity can be measured by using artificial substrates such as para-nitrocatechol sulfate and 4-methylumbelliferyl sulfate or by using the natural substrate 3-O-sulfogalactosylceramide. In some embodiments, ARSA enzyme activity is increased by about 5%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000% or more as compared to a control. In some embodiments, ARSA enzyme activity is increased by at least 1-fold, 2-fold, 3-fold, 4-fold, 5-fold, 6-fold, 7-fold, 8- fold, 9-fold or 10-fold as compared to a control.

In some embodiments, treatment refers to increased stability of ARSA enzyme and its activity in various tissues. In some embodiments, treatment refers to increased stability of ARSA enzyme and its activity in brain target tissues, spinal cord, peripheral nerves and/or other peripheral target tissues. ARSA enzyme stability and its activity can be measured by using artificial substrates such as para-nitrocatechol sulfate and 4-methylumbelliferyl sulfate or by using the natural substrate 3-O-sulfogalactosylceramide. In some embodiments, ARSA enzyme stability is increased by about 5%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1000% or more as compared to a control. In some embodiments, ARSA enzyme stability is increased by at least 1-fold, 2-fold, 3-fold, 4-fold, 5-fold, 6-fold or more as compared to a control, for example the control being the enzyme of SEQ ID NO: 1.

In some embodiments, increased ARSA enzymatic activity (preferably activity over time) is at least approximately 10 nmol/hr/mg, 20 nmol/hr/mg, 40 nmol/hr/mg, 50 nmol/hr/mg, 60 nmol/hr/mg, 70 nmol/hr/mg, 80 nmol/hr/mg, 90 nmol/hr/mg, 100 nmol/hr/mg, 150 nmol/hr/mg, 200 nmol/hr/mg, 250 nmol/hr/mg, 300 nmol/hr/mg, 350 nmol/hr/mg, 400 nmol/hr/mg, 450 nmol/hr/mg, 500 nmol/hr/mg, 550 nmol/hr/mg, 600 nmol/hr/mg or more. In some embodiments, ARSA enzymatic activity is increased in the lumbar region. In some embodiments, increased ARSA enzymatic activity in the lumbar region is at least approximately 2000 nmol/hr/mg, 3000 nmol/hr/mg, 4000 nmol/hr/mg, 5000 nmol/hr/mg, 6000 nmol/hr/mg, 7000 nmol/hr/mg, 8000 nmol/hr/mg, 9000 nmol/hr/mg, 10,000 nmol/hr/mg, or more.

In some embodiments, treatment refers to decreased progression of loss of cognitive ability. In certain embodiments, progression of loss of cognitive ability is decreased by about 5%, 10%, 15%, 20%, 25%, 30%, 35%, 40%, 45%, 50%, 55%, 60%, 65%, 70%, 75%, 80%, 85%, 90%, 95%, 100% or more as compared to a control. In some embodiments, treatment refers to decreased developmental delay. In certain embodiments, developmental delay is de-creased by about 5%, 10%, 15%, 20%, 25%, 30%, 35%, 40%, 45%, 50%, 55%, 60%, 65%, 70%, 75%, 80%, 85%, 90%, 95%, 100% or more as compared to a control.

In some embodiments, treatment refers to increased survival (e.g. survival time). For example, treatment can result in an increased life expectancy of a patient. In some embodiments, treatment according to the present invention results in an increased life expectancy of a patient by more than about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 105%, about 110%, about 115%, about 120%, about 125%, about 130%, about 135%, about 140%, about 145%, about 150%, about 155%, about 160%, about 165%, about 170%, about 175%, about 180%, about 185%, about 190%, about 195%, about 200% or more, as compared to the average life expectancy of one or more control individuals with similar disease without treatment. In some embodiments, treatment according to the present invention results in an increased life expectancy of a patient by more than about 6 month, about 7 months, about 8 months, about 9 months, about 10 months, about 11 months, about 12 months, about 2 years, about 3 years, about 4 years, about 5 years, about 6 years, about 7 years, about 8 years, about 9 years, about 10 years or more, as compared to the average life expectancy of one or more control individuals with similar disease without treatment. In some embodiments, treatment according to the present invention results in long term survival of a patient. As used herein, the term "long term survival" refers to a survival time or life expectancy longer than about 40 years, 45 years, 50 years, 55 years, 60 years, or longer. The terms, "improve," "increase" or "reduce," as used herein, indicate values that are relative to a control. In some embodiments, a suitable control is a baseline measurement, such as a measurement in the same individual prior to initiation of the treatment described herein, or a measurement in a control individual (or multiple control individuals) in the absence of the treatment described herein. A "control individual" is an individual afflicted with the same form MLD (e.g., late-infantile, juvenile, or adult-onset form), who is about the same age and/or gender as the individual being treated (to ensure that the stages of the disease in the treated individual and the control individual(s) are comparable.

The individual (also referred to as "patient" or "subject") being treated is an individual (fetus, infant, child, adolescent, or adult human) having MLD or having the potential to develop MLD. The individual can have residual endogenous ARSA expression and/or activity, or no measurable activity. For example, the individual having MLD may have ARSA expression levels that are less than about 30-50%, less than about 25-30%, less than about 20-25%, less than about 10-15%, less than about 5-10%, less than about 0.1-5% of normal ARSA expression levels.

In some embodiments, the individual is an individual who has been recently diagnosed with the disease. Typically, early treatment (treatment commencing as soon as possible after diagnosis) is important to minimize the effects of the disease and to maximize the benefits of treatment.

A treatment according to the invention preferably comprises the administration of a therapeutically effective amount of the compound of the invention to a subject in need of the treatment.

Preferred embodiments pertain to the treatment which comprises the intravenous, intracerebral, intrathecal and/or intracerebroventricular injection or infusion of a therapeutically effective amount of the compound of the invention to a subject in need of the treatment.

The compounds of the invention for use in therapeutic treatments are administered to a patient suffering from a disorder as mentioned herein, in therapeutically effective doses. As used herein, the term "therapeutically effective dose" intends that dose of ARSA that achieves a therapeutic effect, and is typically in the range of about 0.05 mg/kg/day to about 1.0 mg/kg/day for both children and adults, and more preferably of about 0.075 mg/kg/day to about 0.3 mg/kg/day. The therapeutic dose of compound of the invention can be administered as a single dose or divided doses given in certain intervals of time, for example as two, three, four or more daily doses. The therapeutic dose can also be administered as a continuous infusion into the cerebrospinal fluid (intrathecal or intracerebroventricular infusion) or blood (intravenous infusion) released from a pump, e.g., an implantable miniature pump. A preferred treatment comprises the administration of 0.1 to 1000 mg of mutated ARSA enzyme, or the functional fragment thereof, of the invention to a subject in need of the treatment, for example once a week, once every two weeks, or once every three weeks, for at least 2, preferably 3, 4, 5, 6, 7, 8, 9, 10, 11, or 12 months, or longer.

In some embodiments, the treatment of the invention is a gene therapy or an enzyme replacement therapy. The replacement enzyme suitable for the invention is preferably a mutant ARSA as described herein before. The replacement enzyme suitable for the present invention may be produced by any available means. For example, replacement enzymes may be recombinantly produced by utilizing a host cell system engineered to express a replacement enzyme-encoding nucleic acid. Where enzymes are recombinantly produced, any expression system can be used. To give but a few examples, known expression systems include, for example, egg, baculovirus, plant, yeast, or mammalian cells.

In some embodiments, mutated ARSA enzymes, or the functional fragments thereof, suitable for the present invention are produced in mammalian cells. Non-limiting examples of mammalian cells that may be used in accordance with the present invention include BALB/c mouse myeloma line (NSO/1, ECACC No:85110503); human retinoblasts (PER.C6, Cru-Cell, Leiden, The Netherlands); monkey kidney CV1 line transformed by SV40 (COS-7, ATCC CRL 1651); human embryonic kidney line (293 or 293 cells subcloned for growth in suspension culture, Graham et al, J. Gen Virol, 36:59,1977); human fibrosarcoma cell line (e.g., HT1080); baby hamster kidney cells (BHK, ATCC CCL 10); Chinese hamster ovary cells +/-DHFR (CHO, Urlaub and Chasin, Proc. Natl. Acad. Sci. USA, 77:4216, 1980); mouse Sertoli cells (TM4, Mather, Biol. Reprod., 23:243-251, 1980); monkey kidney cells (CV1 ATCC CCL 70); African green monkey kidney cells (VERO- 76, ATCC CRL-1 587); human cervical carcinoma cells (HeLa, ATCC CCL 2); canine kidney cells (MDCK, ATCC CCL 34); buffalo rat liver cells (BRL 3 A, ATCC CRL 1442); human lung cells (W138, ATCC CCL 75); human liver cells (Hep G2, HB 8065); mouse mammary tumor (MMT 060562, ATCC CCL51); TRI cells (Mather et al, Annals N.Y. Acad. Sci., 383:44-68, 1982); MRC 5 cells; FS4 cells; and a human hepatoma line (Hep G2).

In some embodiments, the mutated ARSA enzymes, or the functional fragments thereof, delivered using a method of the invention contain a moiety that binds to a receptor on the surface of brain cells to facilitate cellular uptake and/or lysosomal targeting. For example, such a receptor may be the cation- independent mannose-6-phosphate receptor (CI-MPR) which binds the mannose-6-phosphate (M6P) residues. In addition, the CI-MPR also binds other proteins including IGF -II. In some embodiments, a replacement enzyme suitable for the present invention contains M6P residues on the surface of the protein. In some embodi-ments, a replacement enzyme suitable for the present invention may contain bis-phosphorylated oligosaccharides which have higher binding affinity to the CI-MPR. In some embodiments, a suitable enzyme contains up to about an average of about at least 20% bis-phosphorylated oligosaccharides per enzyme. In other embodiments, a suitable enzyme may contain about 10%, 15%, 18%, 20%, 25%, 30%, 35%, 40%, 45%, 50%, 55%, 60% bis-phosphorylated oligosaccharides per enzyme. While such bis-phosphorylated oligosaccha-rides may be naturally present on the enzyme, it should be noted that the enzymes may be modified to possess such oligosaccharides. For example, suitable replacement enzymes may be modified by certain enzymes which are capable of catalyzing the transfer of N-acetylglucosaminei-phosphate from UDP-N-acetylglucosamine to the 6' position of alpha-1,2-linked mannoses on lysosomal enzymes. Methods and compositions for producing and using such enzymes are described by, for example, Canfield et al. in U.S. Pat. No. 6,537,785, and U.S. Pat. No. 6,534,300, each incorporated herein by reference.

In some embodiments, mutated ARSA enyzmes for use in the present invention may be conjugated or fused to a lysosomal targeting moiety that is capable of binding to a receptor on the surface of brain cells. A suitable lysosomal targeting moiety can be IGF-I, IGF-II, RAP, apolipoprotein E (as described herein elsewhere), p97, and variants, homologues or fragments thereof (e.g., including those peptide having a sequence at least 70%, 75%, 80%, 85%, 90%, or 95% identical to a wild-type mature human IGF-I, IGF- II, RAP, apolipoprotein E, P97 peptide sequence).

In some embodiments, a therapeutic protein includes a targeting moiety (e.g., a lysosome targeting sequence) and/or a membrane -penetrating peptide. In some embodiments, a targeting sequence and/or a membrane-penetrating peptide is an intrinsic part of the therapeutic moiety (e.g., via a chemical linkage, via a fusion protein). In some embodiments, a targeting sequence contains a mannose-6-phosphate moiety. In some embodiments, a targeting sequence contains an IGF-I moiety. In some embodiments, a targeting sequence contains an IGF-II moiety.

A preferred treatment of a disease of the invention involves gene therapy. Such methods may include the transformation of a human cell with a mutated ARSA and infusion of the so produced cell into a patient according to the above described preferred routes. Preferably gene therapy may comprise obtaining autologous adult stem cells of a patient, preferably HSCs. These cells are in a next step genetically altered to express a mutated ARSA of the invention. Genetically alteration may be achieved by either transforming the cell with an expression vector of the invention, or alternatively, by directly mutating the HSC endogenous ARSA using for example gene editing (e.g. CRISPR/Cas9 approaches). If the endogenous ARSA comprises one or more mutations decreasing ARSA activity and/or expression, the approach also comprises repairing ARSA deficiency by reconstitution of the wild-type sequence at the respective positions. In general the present invention also pertains to methods for generating a mutated ARSA as described before, by providing a target cell which endogenously expresses human ARSA, and introducing the ARSA mutations of the invention into the endogenous human ARSA sequence.

The pharmaceutical compositions according to the invention are in preferred embodiments suitable for CNS delivery of the compounds of the invention.

In **a sixth aspect,** the invention further provides a method for producing a mutated ARSA of the invention, preferably wherein the method comprises a step of recombinantly expressing the mutated ARSA protein.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

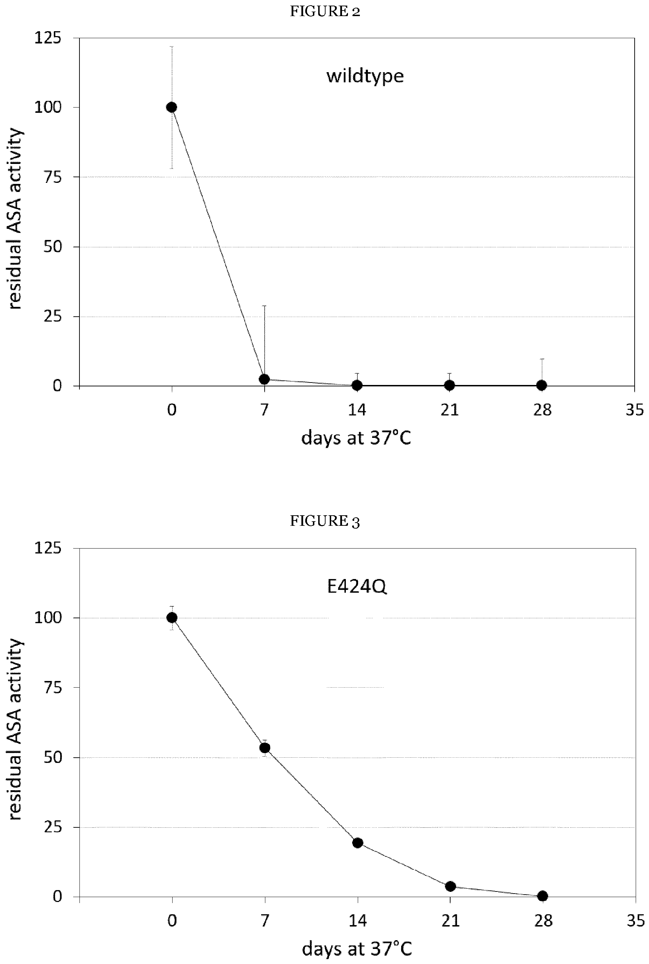

**Figure 1****:** shows the extracellular half-life of human ASA (hARSA) with permutated glutamate-424 (E424). Wildtype hARSA was cloned into the eukaryotic expression plasmid PCDNA3 (Invitrogen, Carlsbad, CA, USA) and E424 was exchanged by all 19 alternative proteinogenic amino acids using site-directed mutagenesis. Then, chinese hamster ovary (CHO) K1 cells (300,000 cells/35 mm dish) were transfected with Turbofect (Thermo Fisher Scientific, Dreieich, Germany) and 4 µg plasmid DNA each. Two days after transfection the conditioned media were harvested and incubated at 37°C. Immediately after harvesting (day 0) and in weekly intervals for up to day 28, hARSA levels were measured with an enzyme-linked immunosorbent assay (ELISA). The half-lives of the individual hARSA-mutants were extracted from the blotted kinetics of decline (see Fig. 2-5 as examples). Data were confirmed by a functional assay measuring the time-dependent decline of ASA activity (not shown).
**Figure 2****:** shows the kinetics of decline of wildtype hARSA in conditioned cell culture medium. For experimental details see Figure 1. The calculated half-life is 4.6 days.
**Figure 3****:** shows the kinetics of decline of the hARSA-mutant E424Q in conditioned cell culture medium. For experimental details see Figure 1. The calculated half-life is 7.5 days

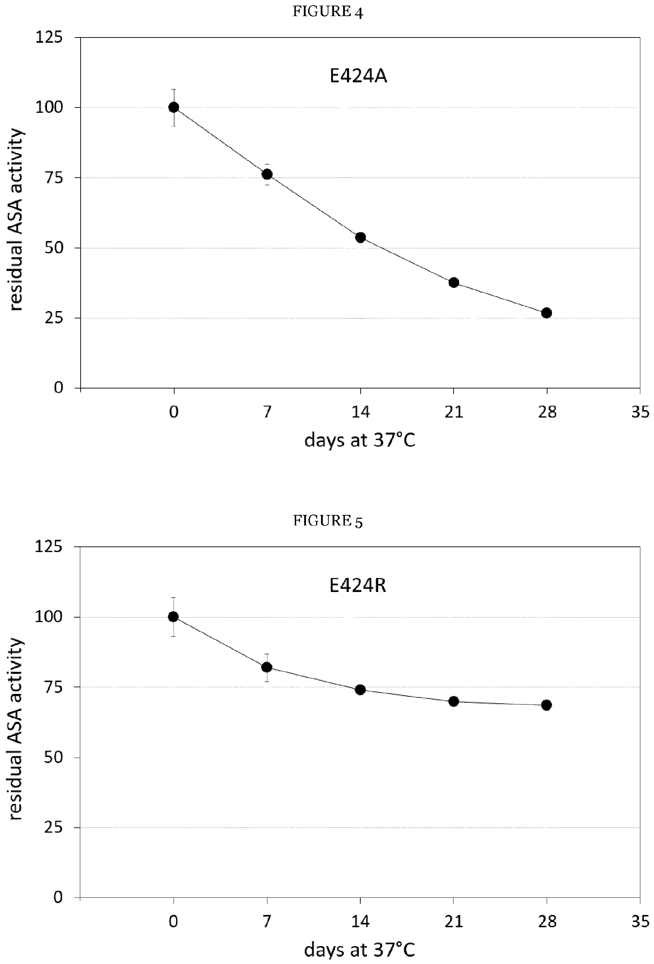

**Figure 4****:** shows the kinetics of decline of the hARSA-mutant E424A in conditioned cell culture medium. For experimental details see Figure 1. The calculated half-life is 15.7 days.
**Figure 5****:** shows the kinetics of decline of the hARSA-mutant E424R in conditioned cell culture medium. For experimental details see Figure 1. The half-life is above 28 days and cannot be precisely calculated from the available data set.

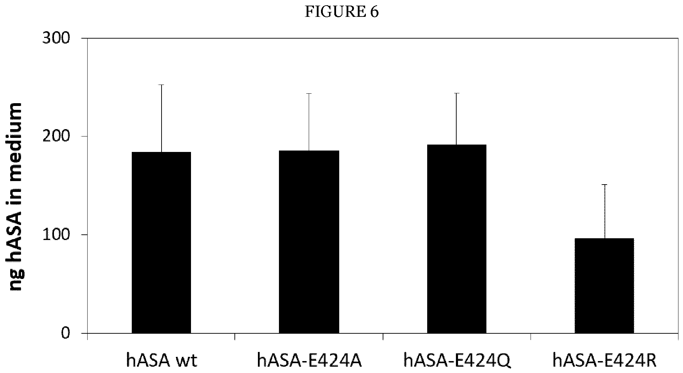

**Figure 6****:** shows the ammonium chloride-induced hypersecretion of wildtype hARSA and three hARSA-mutants. CHO-K1 cells were transfected in triplicates as described in the legend of Figure 1 and ammonium chloride (10 mM) was added to the culture medium 24 h after transfection. This causes a pH shift in the endosomal system of the cells so that newly synthesized hARSA can no longer be targeted to the lysosomal compartment, but is delivered from the cell. The hARSA concentrations in the media were measured by ELISA 24 h after addition of ammonium chloride and are shown as means +/- SD. Mutations that prevent hARSA to adopt its proper three-dimensional structure would be retained in the endoplasmic reticulum (ER) and result in very low extracellular concentrations. This is not the case proving that the hARSA-mutants are correctly folded and overcome the conformational proofreading system of the ER

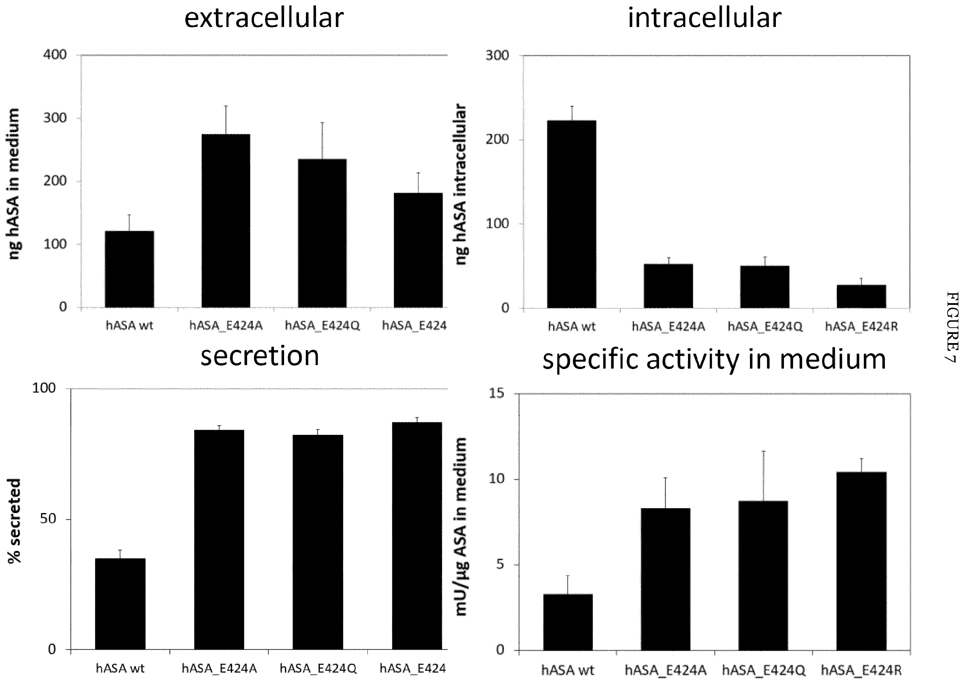

**Figure 7****:** shows the targeting and specific activity of wildtype hARSA and three hARSA-mutants. CHO-K1 cells were transfected as described in the legend of Figure 1. To determine the rate of physiological hARSA-secretion, the conditioned media and the cells were harvested 2 days after transfection and the extra- and intracellular amount of hARSA was measured by ELISA. Bars represent means +/- SDs of n=3 independent transfection experiments. Compared to wildtype hARSA the three hARSA-mutants are less efficiently targeted to the lysosome, but mainly secreted. In addition, the activities of the hARSA-variants were measured in the media by a functional assay. The resulting enzyme units were related to the amount of hARSA (measured by ELISA) yielding the specific activity which is expressed as units hARSA per µg hARSA (U/µg). Compared to wildtype hARSA, the specific activity of all three hARSA-mutants is substantially increased.

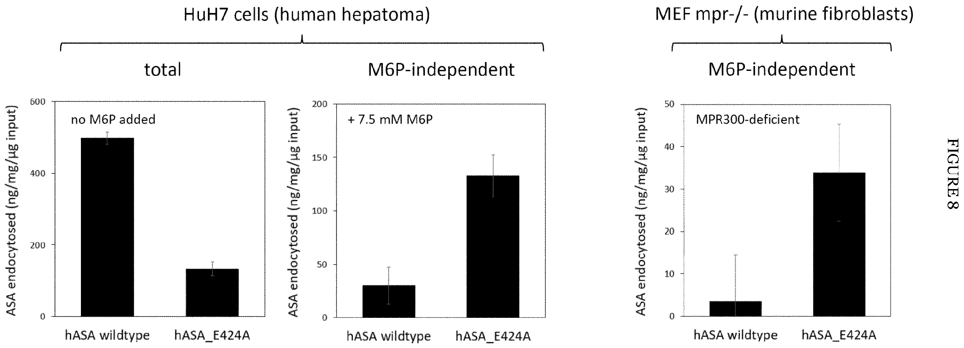

**Figure 8****:** shows the endocytosis of wildtype hARSA and the hARSA-mutant E424A by two types of target cells as indicated. CHO-K1 cells were transfected as described in the legend of Fig. 1. Conditioned medium was collected 48 h later and added to subconfluent cultures of human hepatoma cells and murine fibroblasts, respectively. To prevent uptake via the mannose 6-phosphate (M6P) receptor (MPR300), 7.5 mM soluble M6P was added to some dishes with human hepatoma cells as indicated. After 24 h the cells were harvested by trypsinization and the amount of internalized hARSA and hASA_E424A was determined by ELISA. Human hepatoma cells express MPR300. In the absence of competitive amounts of soluble M6P uptake of hASA_E424A is reduced compared to wildtype hARSA. In the presence of soluble M6P, uptake of the hARSA-mutant is, however, increased. Increased M6P-independent uptake was confirmed by using MPR300-deficient murine fibroblasts as target cells. Bars represent means +/- SDs of n=3 independent transfection experiments per condition.

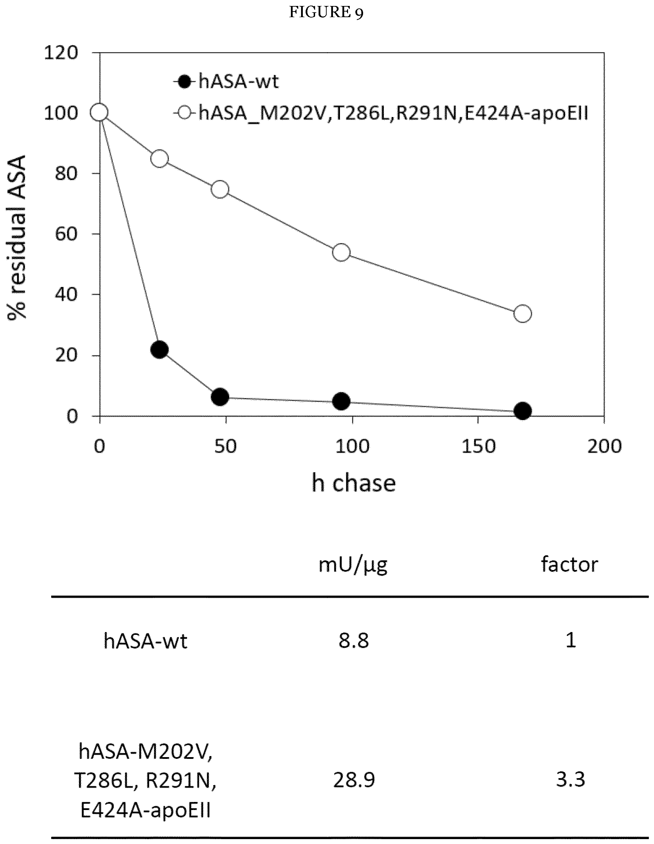

**Figure 9****: A:** Stability of recombinant hARSA and the indicated hARSA-mutant in human blood serum. The two hARSA-variants were recombinantly expressed in CHO suspension cells and purified from the conditioned media by standard procedures. Each hARSA-variant (125 ng) was mixed with 50 µl serum and incubated at 37°C for up to 7 days. The hARSA levels were measured at time point 0 (0 h chase) and after 24, 48, 96 and 168 h. Data are related to the initial ASA level. **B:** Specific activity of hARSA and the indicated hARSA-mutant. The two hARSA-variants were recombinantly expressed in CHO suspension cells, purified from the conditioned media by standard procedures and diluted in an appropriate volume of buffer. The activity and the mass of the ASA-variants was determined by a functional assay and ELISA, respectively. The resulting enzyme units were related to the masses yielding the specific activity which is expressed as units hARSA per µg hARSA (U/µg).

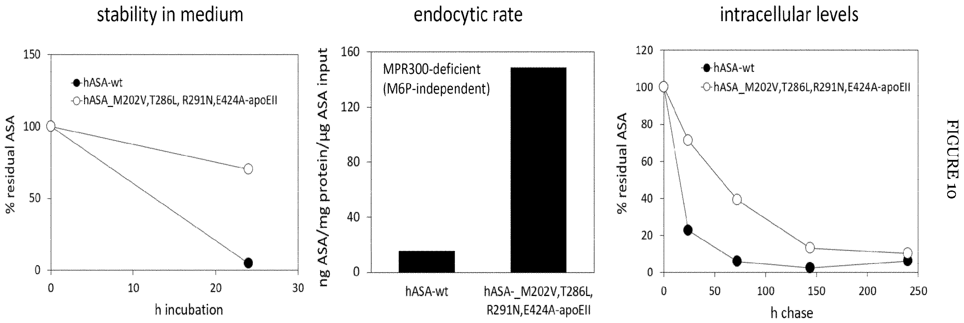

**Figure 10****:** shows the functional parameters of recombinant hARSA and the indicated hARSA-mutant. Recombinantly expressed enzyme was added to the medium of MPR300-deficient murine fibroblasts at a concentration of 5 µg/ml. Cells were allowed to endocytose the hARSA-variants within a 24 h feeding period. After that, cells were washed with PBS and a glycine-buffer pH 3.0 to detach surface-bound enzyme and incubated with fresh medium for up to 10 days. Within this chase period, cells were harvested at different time points and the intracellular concentrations of the hARSA-variants were determined by ELISA. Left: Decline of hARSA levels within the feeding period. Middle: Intracellular concentration of the two hARSA-variants immediately after the feeding period (day 0). Right: time-dependent decline of intracellular levels.

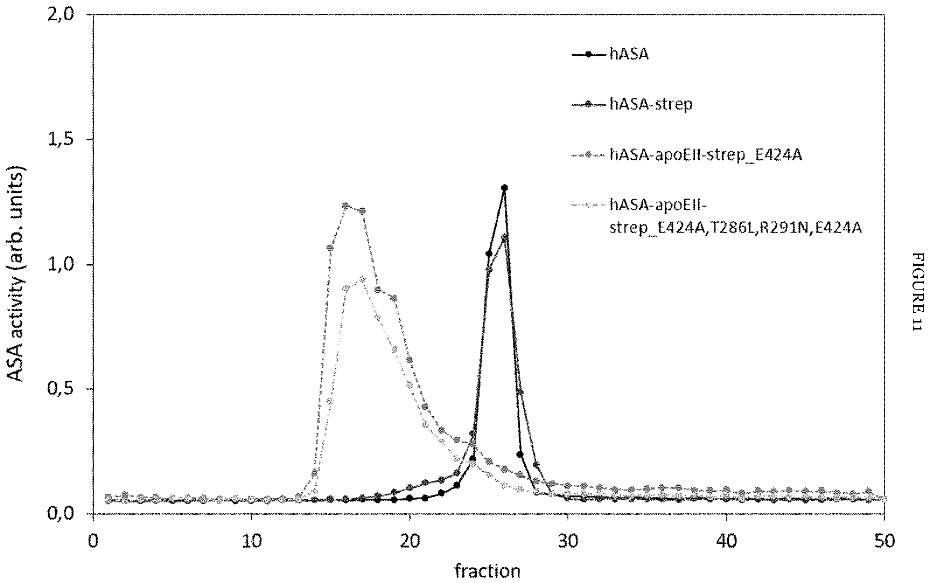

**Figure 11****:** shows an oligomeric state of different hASA-variants at pH 7.0. The indicated hASA-variants were separated by size exclusion chromatography using a Superdex 200 column (Amersham Pharmacia) linked to an Äkta FPLC system (GE Healthcare). The buffer was 150 mM NaCl, 20 mM Bis-Tris, pH 7.0. Fractions (0.5 ml) were collected and analysed on ASA activity using p-nitrocatechol sulfate as a substrate. Elution profiles of the four indicated hASA-variants are superimposed. While wildtype hASA presents as a dimer at neutral pH, E424A-mutants form octamers.

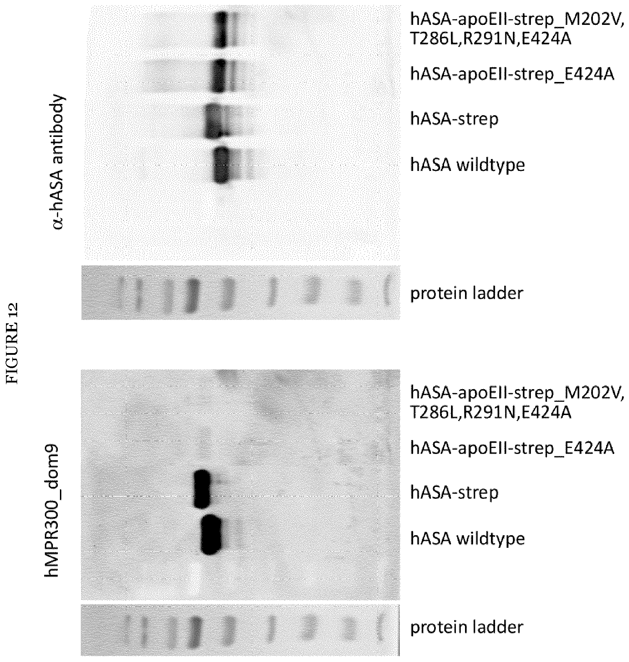

**Figure 12****:** shows a filter binding assay. The indicated hASA-variants (1 µg each) were separated by SDS-PAGE and transferred to a polyvinylidene difluoride (PVDF)-membrane by Western blotting. Left: The membrane was probed with a myc-tagged fragment of the human MPR300 encompassing the M6P-binding domain (domain 9). Fragment bound to ASA was detected by sequential incubation with a murine monoclonal anti-myc antibody, a peroxidase-conjugated goat anti-mouse antibody and an ECL chemiluminescent substrate. Note that the MPR300 fragment does not bind efficiently to the two E424A-mutants. Right: Control to verify loading of similar amounts of the four ASA-variants. MPR300-fragments were washed from the membrane and ASA was detected by sequential incubation with a rabbit polyclonal anti-ASA antiserum, a peroxidase-conjugated goat anti-rabbit antiserum and ECL chemiluminescent substrate.

The sequences show:
**SEQ ID NOs. 1** shows the amino acid sequence of wild type human ARSA protein(isoform 1) including the signal peptide (underlined) and most preferred positions for mutation (bold and underlined): 
10 20 30 40 50
MGAPRSLLLA LAAGLAVARP PNIVLIFADD LGYGDLGCYG HPSSTTPNLD
 
60 70 80 90 100
QLAAGGLRFT DFYVPVSLCT PSRAALLTGR LPVRMGMYPG VLVPSSRGGL
110 120 130 140 150
PLEEVTVAEV LAARGYLTGM AGKWHLGVGP EGAFLPPHQG FHRFLGIPYS
160 170 180 190 200
HDQGPCQNLT CFPPATPCDG GCDQGLVPIP LLANLSVEAQ PPWLPGLEAR
210 220 230 240 250
YMAFAHDLMA DAQRQDRPFF LYYASHHTHY PQFSGQSFAE RSGRGPFGDS
260 270 280 290 300
LMELDAAVGT LMTAIGDLGL LEETLVIFTA DNGPETMRMS RGGCSGLLRC
310 320 330 340 350
GKGTTYEGGV REPALAFWPG HIAPGVTHEL ASSLDLLPTL AALAGAPLPN
360 370 380 390 400
VTLDGFDLSP LLLGTGKSPR QSLFFYPSYP DEVRGVFAVR TGKYKAHFFT
410 420 430 440 450
QGSAHSDTTA DPACHASSSL TAHEPPLLYD LSKDPGENYN LLGGVAGATP
460 470 480 490 500
EVLQALKQLQ LLKAQLDAAV TFGPSQVARG EDPALQICCH PGCTPRPACC

HCPDPHA
**SEQ ID NO: 2** shows the nucleic acid sequence encoding wild type human ARSA (cDNA): 
ATGGGGGCACCGCGGTCCCTCCTCCTGGCCCTGGCTGCTGGCCTGGCCGTTGCCCGTCCGCCC
AACATCGTGCTGATCTTTGCCGACGACCTCGGCTATGGGACCTGGGCTGCTATGGCACCCC
AGCTCTACCACTCCCAACCTGGACCAGCTGGCGGCGGAGGGCTGCGGTTCACAGACTTCTAC
GTGCCTGTGTCTCTGTGCACACCCTCTAGGGCCGCCCTCCTGACCGGCCGGCTCCCGGTTCGG
ATGGGCATGTACCCTGGCGTCCTGGTGCCCAGCTCCCGGGGGGGCCTGCCCCTGGAGGAGGT
GACCGTGGCCGAAGTCCTGGCTGCCCGAGGCTACCTCACAGGAATGGCCGGCAAGTGGCACCT
TGGGGTGGGGCCTGAGGGGGCCTTCCTGCCCCCCCATCAGGGCTTCCATCGATTTCTAGGCAT
CCCGTACTCCCACGACCAGGGCCCCTGCCAGAACCTGACCTGCTTCCCGCCGGCCACTCCTTGC
GACGGTGGCTGTGACCAGGGCCTGGTCCCCATCCCACTGTTGGCCAACCTGTCCGTGGAGGCG
CAGCCCCCCTGGCTGCCCGGACTAGAGGCCCGCTACATGGCTTTCGCCCATGACCTCATGGCC
GACGCCCAGCGCCAGGATCGCCCCTTCTTCCTGTACTATGCCTCTCACCACACCCACTACCCTC
AGTTCAGTGGGCAGAGCTTTGCAGAGCGTTCAGGCCGCGGGCCATTTGGGGACTCCCTGATGG
AGCTGGATGCAGCTGTGGGGACCCTGATGACAGCCATAGGGGACCTGGGGCTGCTTGAAGAG
ACGCTGGTCATCTTCACTGCAGACAATGGACCTGAGACCATGCGTATGTCCCGAGGCGGCTGC
TCCGGTCTCTTGCGGTGTGGAAAGGGAACGACCTACGAGGGCGGTGTCCGAGAGCCTGCCTTG
GCCTTCTGGCCAGGTCATATCGCTCCCGGCGTGACCCACGAGCTGGCCAGCTCCCTGGACCTG
CTGCCTACCCTGGCAGCCCTGGCTGGGGCCCCACTGCCCAATGTCACCTTGGATGGCTTTGACC
TCAGCCCCCTGCTGCTGGGCACAGGCAAGAGCCCTCGGCAGTCTCTCTTCTTCTACCCGTCCTA
CCCAGACGAGGTCCGTGGGGTTTTTGCTGTGCGGACTGGAAAGTACAAGGCTCACTTCTTCAC
CCAGGGCTCTGCCCACAGTGATACCACTGCAGACCCTGCCTGCCACGCCTCCAGCTCTCTGACT
GCTCATGAGCCCCCGCTGCTCTATGACCTGTCCAAGGACCCTGGTGAGAACTACAACCTGCTG
 
GGGGGTGTGGCCGGGGCCACCCCAGAGGTGCTGCAAGCCCTGAAACAGCTTCAGCTGCTCAA
GGCCCAGTTAGACGCAGCTGTGACCTTCGGCCCAGCCAGGTGGCCCGGGGCGAGGACCCCGC
CCTGCAGATCTGCTGTCATCCTGGCTGCACCCCCCGCCCAGCTTGCTGCCATTGCCCAGATCCC
CATGCCTGA
**SEQ ID NO: 3** shows the amino acid sequence of a mutated ARSA with increased enzymatic activity (bold and underlined are mutated sequences compared to wild-type ARSA): 
MGAPRSLLLALAAGLAVARPPNIVLIFADDLGYGDLGCYGHPSSTTPNLDQLAAGGLRF
TDFYVPVSLCTPSRAALLTGRLPVRMGMYPGVLVPSSRGGLPLEEVTVAEVLAARGYLTGMAGK
WHLGVGPEGAFLPPHQGFHRFLGIPYSHDQGPCQNLTCFPPATPCDGGCDQGLVPIPLLANLS
VEAQPPWLPGLEARYVAFAHDLMADAQRQDRPFFLYYASHHTHYPQFSGQSFAERSGRGPFG
DSLMELDAAVGTLMTAIGDLGLLEETLVIFTADNGPELMRMSNGGCSGLLRCGKGTTYEGGVR
EPALAFWPGHIAPGVTHELASSLDLLPTLAALAGAPLPNVTLDGFDLSPLLLGTGKSPRQSLFFY
PSYPDEVRGVFAVRTGKYKAHFFTQGSAHSDTTADPACHASSSLTAHEPPLLYDLSKDPGENYN
LLGGVAGATPEVLQALKQLQLLKAQLDAAVTFGPSQVARGEDPALQICCHPGCTPRPACCHCPD
PHA
**SEQ ID NO: 4** shows the amino acid sequence of a mutated ARSA with increased enzymatic activity and stability according to the invention (bold and underlined are mutated sequences compared to wild-type ARSA; X is any amino acid except glutamate - E): 
MGAPRSLLLALAAGLAVARPPNIVLIFADDLGYGDLGCYGHPSSTTPNLDQLAAGGLRF
TDFYVPVSLCTPSRAALLTGRLPVRMGMYPGVLVPSSRGGLPLEEVTVAEVLAARGYLTGMAGK
WHLGVGPEGAFLPPHQGFHRFLGIPYSHDQGPCQNLTCFPPATPCDGGCDQGLVPIPLLANLS
VEAQPPWLPGLEARYVAFAHDLMADAQRQDRPFFLYYASHHTHYPQFSGQSFAERSGRGPFG
DSLMELDAAVGTLMTAIGDLGLLEETLVIFTADNGPELMRMSNGGCSGLLRCGKGTTYEGGVR
EPALAFWPGHIAPGVTHELASSLDLLPTLAALAGAPLPNVTLDGFDLSPLLLGTGKSPRQSLFFY
PSYPDEVRGVFAVRTGKYKAHFFTQGSAHSDTTADPACHASSSLTAHXPPLLYDLSKDPGENYN
LLGGVAGATPEVLQALKQLQLLKAQLDAAVTFGPSQVARGEDPALQICCHPGCTPRPACCHCPD
PHA
**SEQ ID NO: 5** shows the amino acid sequence of a mutated ARSA with increased enzymatic activity and stability according to the invention (bold and underlined are mutated sequences compared to wild-type ARSA; X is any amino acid except glutamate - E), further including the ApoEII tag (underlined and italic - SEQ ID NO: 6). The C-terminal apoE-II sequence comprises two copies of the sequence SAWSHPQFEK (SEQ ID NO: 7) as direct tandem repeats separated by the glycine- and serine-rich linker sequence GGGSGGGSGG (SEQ ID NO: 8) (other linker sequences will probably work as well). Due to technical reasons an irrelevant serine (S)-residue was incorporated between the C-terminal alanine (A) of the human ASA sequence and the apoE-II tag (the construct will most likely work also without this serine or with other amino acids or amino acid sequences at this position).

MGAPRSLLLALAAGLAVARPPNIVLIFADDLGYGDLGCYGHPSSTTPNLDQLAAGGLRF
TDFYVPVSLCTPSRAALLTGRLPVRMGMYPGVLVPSSRGGLPLEEVTVAEVLAARGYLTGMAGK
WHLGVGPEGAFLPPHQGFHRFLGIPYSHDQGPCQNLTCFPPATPCDGGCDQGLVPIPLLANLS
VEAQPPWLPGLEARYVAFAHDLMADAQRQDRPFFLYYASHHTHYPQFSGQSFAERSGRGPFG
DSLMELDAAVGTLMTAIGDLGLLEETLVIFTADNGPELMRMSNGGCSGLLRCGKGTTYEGGVR
EPALAFWPGHIAPGVTHELASSLDLLPTLAALAGAPLPNVTLDGFDLSPLLLGTGKSPRQSLFFY
PSYPDEVRGVFAVRTGKYKAHFFTQGSAHSDTTADPACHASSSLTAHXPPLLYDLSKDPGENYN
LLGGVAGATPEVLQALKQLQLLKAQLDAAVTFGPSQVARGEDPALQICCHPGCTPRPACCHCPD
PHASSAWSHPQFEKGGGSGGGSGGSAWSHPQFEK

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Any Mutation of human ARSA at position E424 increases enzyme half-life

Substitution of E424 by any other proteinogenic amino acid consistently increases the half-life hARSA in cell culture medium (Figure 1). It can, therefore, be concluded that the presence of glutamate at this specific position has a destabilizing effect that is abrogated if it is exchanged. Interestingly, the destabilizing effect of E424 is also diminished if aspartate, the second proteinogenic amino acid with negatively charged side chain, is inserted (see E424D). Substitution of E424 by proline reduces the amount of hARSA being delivered to the medium possibly due to misfolding of the enzyme and retention in the endoplasmic reticulum (not shown). Still, the half-life of the small amount of secreted mutant is increased (see E424P). According on the extent of stabilization the amino acid substitutions can be divided into three groups. Group-1 comprises substitutions that increase the half-life 1.3 to 2.6-fold, group-2 around 3.0 to 4.5-fold and group-3 more than 5-fold. The amino acids of a group do not share common biochemical properties such as size, lipophilicity or charge. Within group-3, for example, arginine (R) is a positively charged amino acid with large side chain, whereas glycine (G) is uncharged and has a minimal side chain of only one hydrogen atom. Leucine (L) on the contrary, is a non-polar aliphatic amino acid.

The half life of wild-type hARSA in cell culture medium is approximately 4.6 days (see Figure 2). Substitution of E424 by glutamine (Q) increases the half-life to more than 7 days, whereas alanine (A) and arginine (R) increase it to more than 15 and 28 days (Figure 3 to 5), respectively.

### Example 2: Mutated hARSAs are not retained in the endoplasmic reticulum (ER)

Many missense mutations of hASA result in a misfolded enzyme that is retained by the conformational proofreading machinery of the ER. It is, therefore, important to mention that amino acid exchanges at position 424 are well tolerated and do not cause alterations of the enzyme's three-dimensional structure interfering with activity and passage of the ER. This has been investigated in more detail for the three substitutions E424A, E424Q and E424R by blocking the M6P-receptors in the trans Golgi network with ammonium chloride. As a consequence of the receptor failure newly synthesized wildtype hASA passes the ER and Golgi apparatus, but then follows the default pathway of secretory proteins and is released into the medium instead of being delivered to lysosomes. Under the same conditions, a misfolded hASA-mutant that is retained in the ER cannot does not reach the trans Golgi network and can therefore not be secreted resulting in undetectable to very low extracellular enzyme levels. This does, however, not apply to hASA-E424A, -E424Q and -E424R (Figure 6). It can be concluded that the exchanges at position E424 do not cause retention in the ER. The reduced extracellular concentration of hASA-E424R might be due to a lower transfection efficacy rather than to partial ER-retention. Correct folding of the mutants is also indicated by a functional assay (not shown) showing considerable activities of all mutants in the medium of transfected cells (not shown).

### Example 3: Mutated hARSA has increased extracellular stability

Measurements of extra- and intracellular hARSA-levels suggest that substitution of E424 alters the intracellular targeting of newly synthesized hARSA. Around two third of the wildtype hARSA is intracellularly retained under conditions of overexpression (Figure 7). On contrast, only around 20% of the hARSA-mutants hARSA-E424A, -E424Q and -E424R is intracellularly retained. Thus, a higher percentage of mutant enzyme is delivered from the cell. Interestingly, the specific activity (milliunit enzyme activity per µg enzyme mass) of the hypersecreted mutants is substantially increased compared to wildtype hARSA. This is probably due to the stabilizing effect of the substitutions reducing the rate of enzyme degradation in the medium.

### Example 4: Mutated hARSA shows decreased liver uptake and increased M6P-independent BBB transcytosis

Enzyme replacement therapy using intravenous injection of hARSA has the potential to mitigate the MLD-like disease of ASA knockout mice. High enzyme doses are, however, needed. This is due to a preferential uptake of hARSA by hepatocytes. To analyse uptake of hARSA-mutants by liver cells, we incubated the human hepatoma cell line HuH7 with conditioned medium containing hARSA-E424A. Compared to wildtype hARSA uptake was reduced to approximately 20% (Figure 8). This result suggests, that less of the hARSA-mutants might get lost by liver uptake during enzyme replacement therapy. As a consequence, higher enzyme concentrations would persist in the circulation promoting transfer across the blood-brain barrier into the brain parenchyma. A redistribution of enzyme in favour of the brain is expected to result in higher therapeutic efficacy. In the presence of competitive amounts of M6P uptake of wildtype hARSA by HuH7 cells is substantially reduced indicating that cellular uptake is predominantly mediated by the MPR300. Around 5-fold more hARSA-E424A is endocytosed under the same conditions indicating that the mutant gets access to the cell via M6P-independent pathways. This result was confirmed by feeding MPR300-deficient murine fibroblasts. Also, in this case cellular uptake of hARSA-E424A is substantially higher than uptake of wildtype hARSA.

### Example 5: Hyperactive and hyperstable hARSA mutations and APO-EII functionalization can be combined with each other

As shown in Figure 1, substitution of E424 by any other proteinogenic amino acid increases the stability of hARSA in cell culture medium. Following intravenous enzyme replacement therapy hARSA is present in blood serum. To investigate the stabilizing effect in the presence of serum, a hyperactive hARSA-variant (hARSA-M2020V,T286L,R291N) with additional E424A substitution was incubated for 7 days in human serum at 37°C. The half-life of the stabilized mutant was 108 h compared to only 15 h of wildtype hARSA used as a control (Figure 9A). Thus, the half-life in serum was increased by a factor of 7.2. In murine serum the factor of increase was greater than 7.6 (not shown). The data also show that the three amino acid exchanges M2020V, T286L, R291N, mutations previously shown in hyperactivated hARSA-variant, do not neutralize the stabilizing effect of E424A.

To determine if the stabilizing mutation E424A interferes with the hyperactivity of the previously patented hARSA triple mutant hARSA-M2020V,T286L,R291 a hARSA-variant combining all four amino acid substitutions was recombinantly expressed and analysed. As shown previously the specific activity of hARSA-M2020V,T286L,R291N is around 4.7-fold normal. The quadruple mutant with additional E424-exchange displays a specific activity of 3.3-fold normal (Figure 9B). Consequently, introduction of E424A does not abrogate hyperactivity. Taken together, the three mutations increasing the catalytic rate constant and the single mutation increasing the half-life do not neutralize each other and can be combined in one hyperactive and superstable hARSA-variant.

To investigate the intracellular half-life of the hyperactive and superstable hARSA-variant MPR300-deficient murine fibroblasts were fed with hASA-M2020V,T286L,R291,E424A and the degradation of the internalized enzyme was measured over 10 days. Whereas wildtype hARSA used as a control has an intracellular half-life of 15 h, it was 55 h for the hyperactive and superstable hARSA-variant (Figure 10). Thus, the factor of increase is around 3.7-fold. A very similar factor of 3.6 was obtained for CHO-K1 cells (not shown).

In addition, in figure 11, the mutated ARSA of the invention assembles as a protein octamer having therefore 8 identical mutated ARSA monomers. Further, as shown by filter binding assays, the MPR300 cannot bind to ASA-mutants harboring E424A (Fig. 12). This can be ascribed to the lack of M6P-residues. M6P groups are added to the N-glycans of ASA as it passes through the cis-Golgi network by a reaction involving two different enzymes: UDP-N-acetylglucosamine 1phosphotransferase and α-N-acetylglucosamine-1phosphodiester α-N-acetylglucosaminidase. The phosphotransferase recognizes ASA and other soluble lysosomal enzymes via a patch comprising several lysine residues that are correctly spaced relative to each other. While the negative charge of E424 prevents octamerization of ASA at the near neutral pH of the endoplasmic reticulum and Golgi apparatus, E424-mutants are likely to arrive in the Golgi apparatus as octamers. It can be concluded from the known three-dimensional structure of ASA hat the lysine patch is then no longer surface exposed but buried in the octamer. As a consequence, E424-mutants are not recognized by the phosphotransferase and M6P-residues cannot be formed. This hypothesis, however which shall not be understood to restrict the invention in all embodiments, explains not only the lack of MPR300-binding to E424A-mutants in filter binding assays (Fig. 12), but also the hypersecretion of E424-mutants from cells (Fig. 7). The lack of M6P-residues allows retargeting of recombinant enzyme from the M6P-receptors mainly of liver and spleen to the blood-brain barrier and brain cells. To enhance such targeting, the apoEII tag binding to receptors of the low-density lipoprotein receptor family was linked to the E424-mutants. It was shown previously by us that the apoEII-tag further promotes transcytosis across the blood-brain barrier and increases therapeutic efficacy of enzyme replacement therapy in a mouse model of MLD (Böckenhoff et al., 2014, J Neurosci. 2014 Feb 26;34(9):312-9).

The amino acid sequence of an apoEII tagged superstable and superactive ARSA is shown in SEQ ID NO: 5.

## Claims

1. **A mutated arylsulfatase A (ARSA) enzyme,** comprising an amino acid sequence with at least 90%, most preferably at least 99% sequence identity to SEQ ID NO: 1 (human ARSA enzyme), wherein the mutated ARSA enzyme amino acid sequence when aligned to the sequence of SEQ ID NO: 1, comprises at least one mutation which is a mutation or modification of E424 of SEQ ID NO: 1.

2. The mutated ARSA enzyme according to claim 1, wherein at least one mutation is a modification, deletion or substitution of E424.

3. The mutated ARSA enzyme according to claim 1 or 2, wherein the at least one mutation is a substitution with a W, Y, A, F, R, G, L and preferably is E424R, E424G or E424L.

4. The mutated ARSA enzyme according to any one of claims 1 to 3, wherein the mutated ARSA enzyme amino acid sequence when aligned to the sequence of SEQ ID NO: 1, comprises at least one further mutation at amino acid positions 202, 286 and/or 291 of SEQ ID NO: 1.

5. The mutated ARSA enzyme according to any one of claims 1 to 4, wherein the mutated ARSA enzyme amino acid sequence when aligned to the sequence of SEQ ID NO: 1, comprises at least one further mutation selected from M202V, T286R and/or R291N compared to SEQ ID NO: 1, preferably of at least M202V.

6. The mutated ARSA enzyme according to any one of claims 1 to 5, wherein the mutated ARSA has an increased protein half-life compared to a wild-type human ARSA of SEQ ID NO: 1; and/or has a decreases mannose 6-phosphorylation of lysosomal proteins compared to a wild-type human ARSA of SEQ ID NO: 1.

7. The mutated ARSA enzyme according to any one of claims 1 to 6, further comprising a C-terminally attached apoE-II protein.

8. The mutated ARSA enzyme according to any one of claims 1 to 7, comprising compared to SEQ ID NO: 1 the mutations at positions M202, T286, R291 and E424, and a C-terminal covalently attached apoE-II protein.

9. **An isolated nucleic acid,** comprising a nucleotide sequence encoding the mutated ARSA enzyme according to any of claims 1 to 8.

10. **A vector,** comprising the nucleic acid according to claim 9.

11. The vector according to claim 18, which is an expression vector, comprising promoter sequence operably linked to the nucleic acid according to claim 10.

12. **A recombinant cell,** comprising a mutated ARSA enzyme according to any of claims 1 to 8, a nucleic acid according to claim 9, or a vector according to claim 10 or 11.

13. **A pharmaceutical composition,** comprising a mutated ARSA enzyme according to any of claims 1 to 8, a nucleic acid according to claim 9, or a vector according to claim 10 or 11, or a recombinant cell according to claim 12, together with a pharmaceutically acceptable carrier, stabilizer and/or excipient.

14. **A compound for use in the treatment of a disease,** the compound being selected from a mutated ARSA enzyme according to any of claims 1 to 8, a nucleic acid according to claim 9, or a vector according to claim 10 or 11, a recombinant cell according to claim 12, or a pharmaceutical composition according to claim 13.

15. The compound for use according to claim 24, wherein the disease is a disease **characterized by** a pathological insufficiency of endogenous ARSA, such as a leukodystrophy, preferably metachromatic leukodystrophy.

16. **A method for producing the mutated ARSA** enzyme according to any of claims 1 to 16, a nucleic acid according to claim 17, the vector according to any of claims 18 to 20, the recombinant cell according to claim 21 or 22, or a pharmaceutical composition according to claim 23.
